# EUROPEAN PATENT SPECIFICATION

(11) **EP 3 548 634 B1**
(45) Date of publication and mention of the grant of the patent: **24.02.2021**
(21) Application number: 17822167.7
(22) Date of filing: 01.12.2017
(51) Int. Cl.: C12Q 1/6827, C12Q 1/6818, C12Q 1/6823, C12Q 1/6858

(54) **PROBE FOR DETECTION OF SNPS**
SONDE ZUR DETEKTION VON SNPS
SONDE POUR LA DÉTECTION DE SNP

(30) Priority: 02.12.2016 EP 16201908; 02.06.2017 EP 17174285
(43) Date of publication of application: 09.10.2019
(73) Proprietor: CONGEN Biotechnologie GmbH, 13125 Berlin (DE)
(72) Inventor: MERGEMEIER, Steffen, 10243 Berlin (DE)
(74) Representative: Hertin und Partner Rechts- und Patentanwälte PartG mbB
(86) International application number: PCT/EP2017/081195
(87) International publication number: WO 2018/100161

(56) References cited:
- WO-A2-2011/027966

## Description

The invention relates to a hydrolysis probe for use in a real-time PCR for the detection of a single nucleotide polymorphism (SNP) in a nucleic acid target sequence, or essentially any single nucleotide difference between two nucleic acid sequences, and methods and kits employing said probe. The probe of the invention comprises an oligonucleotide sequence complementary to the region of the target nucleic acid sequence comprising said SNP and a pair of interactive labels, at least one label being signal-generating and the labels being effectively positioned on the oligonucleotide to quench the generation of detectable signal, said labels being separated by a nuclease susceptible cleavage site, wherein said probe is blocked at the 3'-end terminus to prohibit incorporation of said probe into a primer extension product, and wherein said oligonucleotide sequence of said probe comprises two adjacent portions, said portions being of equal length or differing by one nucleotide in length, wherein the base complementary to said SNP is located within the first portion of the probe sequence from the 5'-end, and located within the second portion of the probe there is one or more mismatches to the target sequence, or the base complementary to said SNP is located within the second portion of the probe, and located within the first portion of the probe there is one or more mismatches to the target sequence.

### BACKGROUND OF THE INVENTION

In genetic analysis of samples containing nucleic acids, the detection of point mutations or single nucleotide polymorphisms (SNPs) is common *[*Katsanis, S.H. et al. Nature reviews Gentics 14, 415-426 (2013*)].* Point mutations in the human genome are called SNPs and are the most common genetic alteration of the human genome. Point mutations are also of great importance in the genome of microorganisms such as bacteria or viruses. Of particular importance is the development of resistance against antibiotics or other drugs through point mutations.

When working with human genetic material, the scope of detecting SNPs comprises virtually all aspects of the field of personalized medicine. For example, the risk assessment of certain gene variants is of high importance. Factor V Leiden is, for example, a coagulation disorder that is due to a point mutation in the corresponding gene, just like other coagulation disorders (Factor 2 or MTHFR), lactose intolerance, hemochromatosis and many others.

When applying personalized medicine, a number of diagnostic and therapeutic decisions are based on SNP measurements. SNPs are analyzed both in normal DNA as well as in bisulfite treated DNA to detect changes in DNA methylation. "Decision-relevant" SNPs in DNA sequences are, for example, activating mutations in the EGFR gene, which can be treated with specific drugs such as Gefitinib or Erlotinib.

The methylation status of DNA is used as a diagnostic marker for the screening of non-invasive colorectal cancer (as offered, for example, by the company *Exact Sciences* from stool samples or the company *Epigenomics* from blood samples). The determination of the methylation state of the MGMT gene determines the perspective of the patient and the treatment options available for patients suffering from glioblastoma. Because molecular analysis of DNA methylation commonly employs bisulfite treatment and subsequent identification of sequence differences, methods employed for detecting SNPs are highly relevant for DNA methylation analysis.

In microorganisms, point mutations are particularly associated with the development of resistance of microorganisms to antibiotics. For example, ESBL (extended spectrum beta-lactamase) microorganisms have developed through acquisition of a point mutation, which confers resistance to wide range of beta-lactamase antibiotics.

Point mutation analysis is carried out by means of a number of different technologies. Among the most fast, simple and inexpensive fluorescence-based PCR methods for SNP analysis are the 5'-exonuclease assay and the melting curve analysis. However, both methods are associated with technical problems in the evaluation of the measurement results.

When using hybridization probes for melting curve analysis, it is necessary to carry out a determination of melting temperatures after the actual PCR, therefore representing a two-step method, reducing throughput in sample analysis and increasing the likelihood of error. The analysis of the melting curves thus formed is not always easy and especially in the presence of both SNP variants (a target sequence with a SNP and the analogous sequence without the SNP) is commonly problematic.

When performing SNP analysis using a 5'-exonuclease assay other problems may also occur. The 5'-exonuclease probes used are not 100% specific for a SNP variant. Therefore, typically a probe for SNP variant 1 is designed and a second probe for SNP variant 2. The probe for SNP variant 1 is labeled with a dye, e.g. FAM, and the probe for the SNP variant 2 with another dye, e.g. VIC. When performing real-time PCR with both probes, signals are detected in channels specific for each of the two dyes. Therefore, a specific genotyping analysis software has to be used for the evaluation of the 5 'exonuclease-based PCR data. In principle, the software is correlating the fluorescence signals of the two dyes in a x-y-diagram by plotting the signal from one dye on the y-axis, and the signal of the other dye on the x-axis. By means of a standard, the resulting analysis points can be associated with a SNP variant. Although this method is feasible, it is technically complicated, requires specialized software and is plagued with artefacts and difficulties in quantification.

Point mutations in the genome of microorganisms are generally determined by sequencing, which also employs more complicated and costly technology.

WO2011/027966A2 describes hydrolysis probes for use in a PCR for the detection of SNPs. These probes comprise hybridization regions that are sufficiently complementary to a target sequence to hybridize with it under reaction conditions, while they can comprise mismatches to the target sequence. However, there is no specific description of the relative position of the base/nucleotide complementary to the SNP in comparison to the additional mismatches of the probe to the target sequence in order to increase specificity of SNP detection.

In light of the prior art there remains a significant need in the art to provide additional means for detecting SNPs in genetic material. It would be desirable to find a simple technical solution that can distinguish between SNP variants by standard techniques available in any molecular biology laboratory, such as real-time PCR analysis, which would provide an unambiguous result without the need for additional analysis tools and lengthy result evaluation.

### SUMMARY OF THE INVENTION

In light of the prior art the technical problem underlying the present invention is to provide alternative and/or improved means for detecting and analyzing SNP variants in nucleic acids.

This problem is solved by the features of the independent claims. Preferred embodiments of the present invention are provided by the dependent claims.

The invention therefore relates to a probe for use in a real-time PCR for the detection of a SNP in a nucleic acid target sequence, said probe comprising an oligonucleotide sequence complementary to a region of a nucleic acid target sequence comprising said SNP, and a pair of interactive labels, at least one label being signal-generating and the labels being effectively positioned on the oligonucleotide to quench the generation of detectable signal, said labels being separated by a nuclease susceptible cleavage site, wherein said probe is blocked at the 3'-end terminus to prohibit incorporation of said probe into a primer extension product, and wherein said oligonucleotide sequence of said probe comprises two adjacent portions, said portions being of equal length or differing by one nucleotide in length, wherein the base complementary to said SNP is located within the first portion of the probe sequence from the 5'-end, and located within the second portion of the probe there is one or more mismatches to the target sequence, or the base complementary to said SNP is located within the second portion of the probe, and located within the first portion of the probe there is one or more mismatches to the target sequence.

As used herein, the terms "probe" and "hydrolysis probe" may be used interchangeably.

The probe thereby enables SNP detection with reduced false positive signal. When a probe (not of the invention) has a 100% identity to the target sequence with the SNP, signal will be generated in the amplification reaction when the SNP is present. When the SNP is not present, some background signal will also be produced, as only one mismatch (i. e. only the missing SNP is not complementary to the probe) is evident between probe and target. However, when using a probe of the invention, comprising one or more (preferably one) mismatch to the target sequence, the probe leads to signal production in the amplification reaction when the SNP is present, and essentially no false positive signal when the SNP is not evident, due to the presence of two (or more) mismatches between probe and target (i. e. the missing SNP is not complementary to the probe and there is a further intentional mismatch).

It was entirely surprising that a probe containing one or more mismatches to the target sequence provides a higher specificity for the respective SNP as compared to a probe normally used in a 5'-exonuclease assay for the detection of a SNP, which does not contain any mismatches to the target sequence in addition to said SNP.

Under standard conditions it would be expected by a skilled person that mismatches to the sequence lead to a decreased specificity of the probe, which is why it was unexpected that a probe according to the present invention has a higher specificity and generates more reliable results than a probe not containing any mismatches, or at least comparable results.

In particular, the mismatches reduce or eliminate cross-hybridization between the probe and sequences without the SNP, thereby providing a more effective determination of said SNP by reducing false positive signal. Through the higher specificity of the probe according to the present invention it is ensured that the probe is only generating a signal, if the respective SNP is present in the target sequence of the analyzed genetic material, which greatly simplifies the analysis of the SNP detection. This is not the case for a standard probe not containing any additional mismatches.

A standard probe would in many cases also generate a signal if the respective SNP is not present in the target sequence of the genetic material analyzed, although the signal would be lower as compared to the signal generated in presence of genetic material containing the SNP. When using such a standard probe, a clear result can only be generated by comparing the signals generated by two probes detecting different variants of the SNP. Such analysis requires additional software and can lead to difficulties in interpreting RT-PCR data.

In contrast, a probe according to the present invention essentially only generates a signal if the respective SNP, which is complimentary to the oligonucleotide sequence of the probe, is present in the analyzed genetic material. If using a probe according to the present invention, no comparison to another probe encoding a different SNP variant is needed to generate a clear result. The result can be obtained by a simple standard analysis of a real-time PCR reaction, in which a probe according to the present invention is used as a specific reporter probe. If the SNP is present in the target sequence of the probe in the analyzed nucleic acid material, a signal will be generated during the real-time PCR run. If the SNP is not present, essentially no signal will be generated, as in such a case preferably two mismatches are evident between probe and target sequence.

It was entirely surprising that the introduction of preferably one mismatch leads essentially to the absence of hybridization of the probe of the present invention, if the SNP to be detected is not present, leading to at least two mismatches between the probe and the target sequence that does not comprise the SNP. Accordingly, the hydrolysis probe of the invention will not be cleaved during the RT-PCR reaction by the polymerase through the exonuclease activity of the polymerase and no signal will be generated.

The present invention is therefore characterized by the entirely surprising finding, that a single intentionally integrated mismatch in the probe sequence compared to the target sequence has essentially no effect on effective hybridization to the target and effective signal generation in a nucleic acid amplification reaction, whereby one additional mismatch leads essentially to a strong, if not almost complete, reduction in signal when assessed using a nucleic acid amplification reaction. This effect is not obvious, as it does not correspond to common assumptions or calculations of interaction energies between two (single stranded) nucleic acid sequences.

Typical estimations of hybridization efficiency between two (single stranded) nucleic acid sequences would indicate a step-wise and predictable reduction in hybridization efficiency with increasing numbers of mismatches. The present invention however dispels this assumption, as it appears that a single mismatch has little (if any) negative effect on hybridization and/or signal production during amplification and that a second additional mismatch leads to substantial interference in hybridization and/or signal production during amplification in the reaction, such that essentially no or very little signal is generated. This phenomenon appears therefore related in some preferred embodiments essentially to an "on/off' signal generation phenomenon between probe-template pairs that exhibit one mismatch or two mismatches, respectively.

Surprisingly, this on/off signal generation phenomenon seems to be beneficially associated with the presence of a blocked 3'-end of the probe of the present invention, since in essentially all tested examples of probes that lack the blocked 3'-end and, but are otherwise identical to the probes of the present invention, the clear cut on/off signal generation was not present anymore and even probes that have two mismatches to the target sequence could in some cases generate a detectable signal. Accordingly, the 3'-prime block seems to unexpectedly influence the binding activity and/or the signal-generating properties of the probe. The same phenomenon could be observed in probes that differ from the probes of the invention only by the fact that they only carry one label instead of a pair of interacting labels.

Besides the advantages of simplifying an SNP analysis and generating more reliable results, using a probe according to the present invention containing one or more mismatches to the target sequence for the detection of a SNP is also more cost efficient as compared to using a standard probe without additional mismatches, as only one probe is required for the analysis, and the time needed for performing the analysis and analyzing the data is much shorter, thereby reducing labor costs.

Furthermore, it is entirely surprising to use a probe comprising one or more mismatches to the target sequence that additionally comprises a blocked 3'-terminus that prohibits incorporation of said probe into a primer extension product. In the art, the use of hybridization probes comprising one or more mismatches to the target sequence in the context of an RT-PCR is completely unknown and unexpected. Accordingly, a skilled person would not have thought of designing such a probe and additionally including a block of the 3'-end of the probe. Even in the case that probes comprising additional mismatches would be known in the art for other applications, the use of a 3'-block of the probe would be non-obvious to a skilled person, since such a modification is only useful in the context of a RT-PCR reaction.

Moreover, the probe of the present invention comprises a pair of interactive labels, wherein one label is quenching the generation of a signal by the other label, which leads to the prevention of the generation of a signal. Although the use of such labels has is well established for qPCR probes, the use of a pair of interactive labels in the context of a probe that comprises one or more mismatches has so far not been described. A person skilled in the art would not have designed such a probe in light of the prior art, because probes comprising pairs of interactive labels are mainly or even exclusively used in the context of RT-PCR. However, in the state of the art, RT-PCR hydrolysis probes comprising one or more mismatches have been considered disadvantageous or non-functional. Therefore, it is unexpected to combine these two features of the probe of the present invention.

Normally, when designing a hydrolysis probe for detection of a target signal by RT-PCR, a person skilled in the art would aim to have a complete sequence homology between the sequence to be detected and the hydrolysis probe. It has been observed in the art, that mismatches lead to a decreased or weakened binding of hydrolysis probes, resulting in increased melting temperatures. However, it remained hard to predict whether a probe would bind to a target sequence, if one or more mismatches were included, and at what temperature this would occur. Even when using complicated formulas for calculating or estimating binding and binding temperatures that take into account the kind of mismatches and the length of the probes it remained uncertain, if the calculated predictions were reliable. Additionally, the application in RT-PCR methods involving rapid cycling through different reaction temperatures requires reliable and quick binding of hydrolysis probes. Therefore, a skilled person would not have designed a hydrolysis probe for RT-PCR application that intentionally comprises one or more mismatches, because it would have been assumed that such mismatches make the method unreliable an error prone. Accordingly, the development of the hydrolysis probe of the present invention for application in the context of a RT-PCR is entirely surprising and the observed advantages are completely unexpected.

Surprisingly, it is apparent that probes of the present invention that comprise corresponding pairs of a signal-generating and signal-quenching labels in combination with a blocked 3'-end are extremely stable and can be stored at room temperature without degradation of the probe for extremely long periods.

In a preferred embodiment of the invention, said one or more mismatches to the target sequence in the oligonucleotide sequence of the probe are C-A, C-T or A-A mismatches. In a preferred embodiment the mismatches to the target sequence are C-A or C-T mismatches. These mismatches relate to the mismatch formed between the bases of the probe and target, respectively, which are aligned with each other due to the flanking complementary sequences between the probe and target.

A probe containing such mismatches displays even greater specificity for detecting a SNP within the respective target sequence as compared to another probe according to the present invention containing other mismatches. It was entirely surprising, that this particular kind of mismatch to the target sequence influences specificity of the probe.

Most preferred are embodiments when one mismatch is present in the probe. In other embodiments two, three or four, or more, mismatches may be present.

The hydrolysis probe of the invention comprises an oligonucleotide sequence that comprises two adjacent portions, said portions being of equal length or differing by one nucleotide in length, wherein the base complementary to said SNP is located within the first portion of the probe sequence from the 5'-end, and said one or more mismatches are located within the second portion of the probe. Alternatively, the base complementary to said SNP is located within the second portion of the probe, and said one or more mismatches are located within the first portion of the probe. In other words, the base complimentary to said SNP and said one or more mismatches are located in different but adjacent regions of the oligonucleotide sequence of the probe, so that the base complementary to said SNP is either located closer to the 5'-end of the probe, whereas said one or more mismatches are located closer to the 3'-end of the probe, or the other way around.

For the present invention the two adjacent portions are not characterised by particular or differing structural features or linkers, but rather represent portions of the sequence identified in order to define the position of the bases complementary to the SNP or the mismatched bases(s).

Surprisingly, this design of the probe oligonucleotide sequence leads to a higher specificity as compared to a probe with different positioning of the base complimentary to said SNP and said one or more mismatches.

In a preferred embodiment of the invention, the base complementary to said SNP is located within a central part of said first portion, wherein said central part consists essentially of a sequence comprising half of the nucleotides of the first portion, and said one or more mismatches are located within a central part of said second portion, wherein said central part consists essentially of a sequence comprising half of the nucleotides of the second portion. Alternatively, the base complementary to said SNP is located within said central part of said second portion, and said one or more mismatches are located within said central part of said first portion.

For the present invention the central part represents a portion of the sequence identified in order to define the position of the bases complementary to the SNP or the mismatched bases(s). The central part of the first portion of the oligonucleotide sequence preferably essentially consists of the second and third eighth of the oligonucleotide sequence of the probe, when dividing the oligonucleotide sequence in eight essentially equal parts and numbering from the 5'-end, and comprises or consists essentially of a quarter of the nucleotides of the sequence. The central part of a portion is preferably positioned essentially in the middle of the portion. Accordingly, the central part of the second portion of the oligonucleotide sequence essentially consists of the sixth and seventh eighth of the oligonucleotide sequence of the probe and also comprises essentially a quarter of the nucleotides of the sequence.

It was entirely surprising that a probe according to these design criteria showed even a higher degree of specificity for detection of a respective SNP.

In another preferred embodiment of the invention, the base complementary to said SNP is located between position 2 and 10 of the probe oligonucleotide sequence from the 5'-end, and said one or more mismatches are located between position 11 and the 3'-end of the probe. Alternatively, the base complementary to said SNP is located between position 11 of the probe sequence from the 5'-end and the 3'-end of the probe, and said one or more mismatches are located between position 2 and 10 of the probe.

It was entirely surprising that a probe according to these design criteria showed even a higher degree of specificity for detection of a respective SNP.

In another preferred embodiment of the invention, the base complementary to said SNP is located between position 4 and 8 of the probe sequence from the 5'-end, and said one or more mismatches are located between position 13 and 19 of the probe. Alternatively, the base complementary to said SNP is located between position 13 and 19 of the probe sequence from the 5'-end, and said one or more mismatches are located between position 4 and 8 of the probe.

It was entirely surprising that a probe according to these design criteria showed even a higher degree of specificity for detection of a respective SNP.

Preferably, a hydrolysis probe according of the invention has a length of 16 to 32 nucleotides, preferentially 18 to 24 nucleotides.

In preferred embodiments of the invention, the base complementary to said SNP is located between position 2 and 12, 3 and 11, 4 and 10, 5 and 9, 6 and 8, or at position 7 of the probe sequence from the 5'-end, and said one or more mismatches are located between position 11 and 23, 12 and 22, 13 and 21, 14 and 20, 15 and 19, 16 and 18, or at position 17 of the probe.

In a preferred embodiment of the invention, one label is a fluorophore and the other label is a quencher, which interacts with said fluorophore.

Any one or more of various fluorophores (e.g. 6-carboxyfluorescein, acronym: FAM, or tetrachlorofluorescein, acronym: TET) and quenchers (e.g. tetramethylrhodamine, acronym: TAMRA) are available and known to a skilled person and can be used in a probe according to the present invention. The pairs of fluorophores and quenchers positioned on one probe are preferably selected, so the quencher molecule quenches the fluorescence emitted by the fluorophore when excited by a light source via FRET (Fluorescence Resonance Energy Transfer). As long as the fluorophore and the corresponding quencher are positioned in proximity on the probe, quenching essentially inhibits any fluorescence signals.

If the probe anneals to the target sequence present in a sample containing genetic material (nucleic acids, preferably DNA) while a nucleic acid region comprising the target sequence comprising the SNP is amplified in a PCR reaction using a polymerase with 5' to 3' exonuclease activity, e.g. Taq polymerase. The primers used for the amplification of said region are selected so that each oligonucleotide primer anneals to its complementary template upstream of any probe annealed to the same nucleic acid strand. During the amplification reaction the 5' to 3' exonuclease activity of the polymerase degrades the probe that has annealed to the template via the nuclease susceptible cleavage site. Degradation of the probe releases the fluorophore from it and breaks the close proximity to the quencher, thus relieving the quenching effect and allowing fluorescence of the fluorophore. Hence, fluorescence can be detected by a suitable detector in the machine used to perform the quantitative PCR, i. e. in a thermal cycler. The fluorescent signal is directly proportional to the amount of fluorophore released from the probe and the amount of nucleic acid template containing the nucleic acid target sequence comprising the SNP.

An advantage of probes labeled with fluorophores or fluorescent dyes and a corresponding quencher is that the different probes can be labeled with different, distinguishable reporter dyes. By using probes labeled with different fluorescent reporters, amplification of two or more distinct sequences can be detected in a single PCR reaction. Multiplex reactions are therefore also envisaged.

In a preferred embodiment of the present invention, said nucleic acid target sequence is obtained and/or derived from a microorganism. The detection of SNPs or point mutations in the genome of microorganisms such as bacteria or viruses is of great importance, especially in cases where such point mutations lead to the development of resistance against antibiotics or other drugs. The present invention provides the means for an improved, simplified and more reliable method to detect such point mutations. Therefore, it will help to detect such point mutations more rapidly. This will help to, for example, improve therapeutic intervention in case of microbial infections with microorganisms that have acquired drug resistance through point mutations.

It was surprising, that the probes according to the present invention can be used for the analysis of any nucleic acid target sequence of microbial origin and therefore represent a universal tool for the analysis of SNPs and point mutations in genetic material from microorganisms.

In another preferred embodiment of the present invention said nucleic acid target sequence is obtained and/or derived from a mammal. The detection of SNPs or point mutations in mammalian genomic material, especially human genetic material, is of great importance. A SNP is a variation in a single nucleotide that occurs at a specific position in the genome, where each variation is present to some appreciable degree within a population.

SNPs commonly underlie differences in mammal's susceptibility to disease; a wide range of human diseases, e.g. sickle-cell anemia, β-thaiassemia and cystic fibrosis result from SNPs. The severity of illness and the way our body responds to treatments are also manifestations of genetic variations. For example, a single base mutation in the APOE (apolipoprotein E) gene is associated with a higher risk for Alzheimer's disease.

Variations in the DNA sequences of humans can affect how humans develop diseases and respond to pathogens, chemicals, drugs, vaccines, and other agents. SNPs are also critical for personalized medicine. Therefore, testing and analysis of mammalian, especially human genetic material for the occurrence of a certain SNP is becoming more important. It was surprising, that a probe according to the present invention is more specific for the detection of a SNP in mammalian genetic material as compared to a standard probe without mismatches. Also, it was unexpected that probes according to the present invention work on genetic material from all kinds of mammals.

Another advantage of the present invention is that due to the increased specificity of the probes according to the present invention, it is possible to generate reliable data about the occurrence of a SNP on one or multiple chromosomes of mammalian genetic material by using two or more probes that are specific for different versions or alleles of a SNP, which are marked by different fluorophore/quencher pairs.

In another preferred embodiment of the present invention, the SNP is an activating mutation. Activating mutations are gain-of-function mutations that change the gene product such that its effect becomes stronger (enhanced activation) or even is superseded by a different and abnormal function. In mammals and humans, such mutations are often associated with diseases including cancer, and detection of such mutations is highly relevant for diagnosis and decision-making concerning treatment options. The present invention therefore facilitates detection of activating point mutations in genetic material and represents an improved technology, which can be used for the development of companion diagnostics.

In a preferred embodiment the present invention the SNP in the nucleic acid target sequence has been introduced by bisulfite treatment of the sample comprising the nucleic acid sequence and successive amplification of the nucleic acid target sequence.

Treatment of DNA with bisulfite converts cytosine residues to uracil, but leaves 5-methylcytosine residues unaffected. Therefore, DNA that has been treated with bisulfite retains only methylated cytosines. Thus, bisulfite treatment introduces specific changes in the DNA sequence that depend on the methylation status of individual cytosine residues, yielding single-nucleotide resolution information about the methylation status of a segment of DNA.

Probes according to the present invention can be used to retrieve this information concerning the methylation status of specific cytosine residues in genetic material. This can be of high relevance for the detection of changed methylation status of cytosine residues, which can be involved in the generation of pathologies, including cancer. In comparison to standard probes without mismatches to the target sequence, the probes according to the present invention ensure higher specificity and a simplified analysis of the results when analyzing bisulfite treated nucleic acids. It was surprising that the probes according to the present invention generate more specific results also when analyzing bisulfite treated nucleic acid sequences.

In another preferred embodiment of the invention, the oligonucleotide sequence of said probe comprises or consists of any one of the oligonucleotide sequences SEQ ID No. 1 to 15.

SEQ ID No. 1 (AGTATAGCGATAGTAATTCAGGTTTTGA) is specific for the human gene encoding CD95L and detects a methylated C on bisulfite-treated DNA. A probe according to the present invention comprising or consisting of SEQ ID No. 1 can be used in a companion diagnostic for a glioblastoma medicament.

The present invention therefore relates to a method comprising the use of the probe as described herein. The method may further comprise diagnosis of a medical conditions, or therapy control of a particular ongoing treatment.

SEQ ID No. 2 (CTGATTAGAATACTTTACTCCACTTAATTAA) and SEQ ID No. 3 (ATTCCAATCAGTGTTATTTCGTTACT) are specific for the rs2032583 T-allele and C-allele of the human gene encoding the ABCB1-transporter, respectively. SEQ ID No. 4 (CATACCATTTATGTCTCTTTCGTCTC) and SEQ ID No. 5 (AAATGTTATGTTTGTTTCGTGGTG) are specific for the rs2235015 G-allele and T-allele of the human gene encoding the ABCB1-transporter, respectively. Probes according to the present invention comprising or consisting of SEQ ID No. 2 to 5 can be used in a companion diagnostics to detect SNP-variants. The four different probes can be used in a single 4-plex PCR reaction, if coupled to distinguishable fluorophore/quencher pairs.

SEQ ID No. 6 (CGTCCACCCAACTTATCTTCAG) and SEQ ID No. 7 (CCGGTAAGCGTGGATCTCG) are specific for the beta-lactamase-genes TEM-39 and TEM-240, respectively, and probes according to the present invention comprising or consisting of SEQ ID No. 6 or 7 can be used for detecting mutations inducing ESBL (extended spectrum beta-lactamases) by real-time PCR.

SEQ ID No. 8 (ACCTGGCACGTAAATCTCTGC) and SEQ ID No. 9 (ATACGTACCAGGTGCAGCACC) are specific for the C-allele and the A-allele of the human HFE-gene, respectively. Two different probes according to the present invention, one comprising or consisting of SEQ ID No. 8 and the other comprising or consisting of SEQ ID No. 9, which are labeled with distinguishable fluorophore/quencher pairs, can be used in a single real-time PCR reaction for the detection of the C282Y mutation in the nucleic acid target sequence to diagnose haemochromatosis, which is induced by this point mutation.

SEQ ID No. 10 (CATGACGACCTATGATGCTAGGTTT) and SEQ ID No. 11 (TAGGTCTTCATGCTTACGGGGA) are specific for the C-allele and the T-allele of the human *IDH1-gene,* respectively. Two different probes according to the present invention, one comprising or consisting of SEQ ID No. 10 and the other comprising or consisting of SEQ ID No. 11, which are labeled with distinguishable fluorophore/quencher pairs, can be used in a single real-time PCR reaction for the detection of a cancer-relevant point mutation in the IDH1-gene. Such probes can be useful for the development of companion diagnostics.

SEQ ID No. 12 (AGCAAGCCTCAACGCTCC) and SEQ ID No. 13 (AGGCTCGCTGAGAGCCACTT) are specific for the mutant and the WT allele of the human *factor II*-gene (gene encoding the clotting factor II, also called prothrombin), respectively. Two different probes according to the present invention, one comprising or consisting of SEQ ID No. 12 and the other comprising or consisting of SEQ ID No. 13, which are labeled with distinguishable fluorophore/quencher pairs, can be used in a single real-time PCR reaction for the detection of factor II mutations associated with coagulation problems and/or blood clotting.

SEQ ID No. 14 (AGGCAAGGAATACAGGCATTT) and SEQ ID No. 15 (TTCCTCGCCTGTCCAAGGA) are specific for the mutant and the WT allele of the human *factor V*-gene (gene encoding factor V, a cofactor of the coagulation, also called proaccelerin or labile factor), respectively. Two different probes according to the present invention, one comprising or consisting of SEQ ID No. 14 and the other comprising or consisting of SEQ ID No. 15, which are labeled with distinguishable fluorophore/quencher pairs, can be used in a single real-time PCR reaction for the detection of factor V mutations associated with Factor V Leiden thrombophilia.

In another preferred embodiment the present invention relates to a pair of probes or hydrolysis probes for use in a nucleic acid amplification reaction, such as a real-time PCR, for the detection of a SNP in a nucleic acid target sequence, wherein
- a first probe or hydrolysis probe is a probe according to the present invention, and
- a second probe or hydrolysis probe is a probe according to the present invention comprising the complementary oligonucleotide sequence as the first probe, with the exception that in place of the base that is complementary to the SNP, the second probe comprises a base that is complementary to the corresponding base of a nucleic acid target sequence that does not comprise said SNP, and
- the signals obtained from the fluorophores of the two probes can be distinguished from each other, preferably through a semi-quantitative or quantitative method.

If the SNP represents a point mutation, for example in a nucleic acid target sequence derived from a microorganism, the second probe comprises a base that is complementary to the corresponding base of the nucleic acid target sequence that corresponds to the un-mutated wild-type sequence. If the SNP represents a variation in a single nucleotide that occurs at a specific position in the genome, for example of a mammal or a human, where each variation is present to some appreciable degree within a population in mammalian, the second probe comprises a base that is complementary to the corresponding base of the nucleic acid target sequence that corresponds to a different allele of the nucleic acid target sequence. If the SNP represents a point mutation that has been introduced by bisulfite treatment of the sample comprising the nucleic acid sequence and successive amplification of the nucleic acid target sequence, the second probe comprises a base that is complementary to the corresponding base of a nucleic acid target sequence that does not comprise said SNP and corresponds to the un-mutated sequence.

By using two distinguishable probes, one of which is specific for the SNP and the other is specific for the un-mutated sequence or the predominant allele of a nucleic acid that may contain the SNP, it is possible to determine the presence of one or both of the target sequences of the two probes and semi-quantitatively or quantitatively determine the ratio of the two target sequences in the tested sample. For example, when performing a real-time PCR reaction for the detection of a SNP using a pair of probes according to the present invention and a sample that does only contain the target sequence of the first probe, only a signal from the first probe will be detected during the reaction, and the other way around.

If the sample contains equal amounts of the target sequence of the first probe as well as of the second probe, for example, when the sample is derived from a patient that is heterozygous for the tested SNP, both signals generated by each of the probes will be equal. If 90 % of the genetic material in the sample comprises the target sequence of the first probe and 10% the target sequence of the second probe, for example, when the sample has been generated by bisulfite treatment and successive amplification of the nucleic acid target sequence to determine the methylation status of a certain cysteine, the ration of the signals generated from the first and the second probe will be 9:1.

Such a semi-quantitative or quantitative analysis would not be possible with standard probes not containing any mismatches to the target sequence, as these are not as specific as the probes according to the present invention and unspecific signals would prevent a semi-quantitative or quantitative analysis. It is surprising that the introduction of one or more mismatches in the oligonucleotide sequence of a probe is allowing the semi-quantitative or quantitative determination of genetic variants in a sample, which would otherwise require substantial additional technical effort.

A further aspect of the invention relates to a method for the detection of a SNP in a nucleic acid target sequence comprising
- the amplification of a region of the nucleic acid target sequence comprising the SNP by a nucleic acid amplification reaction, preferably real-time PCR, employing a reporter probe,
- using one or more probes according to the present invention as a reporter probe for the real-time PCR, and
- detecting and/or measuring the signal generated by the hydrolysis of the probe.

Further, the invention relates to a method for the detection of a SNP in a nucleic acid target sequence comprising:
- amplification of a region comprising the nucleic acid target sequence comprising the SNP by real-time PCR employing one or more reporter probe(s),
- wherein the one or more reporter probe(s) comprise one or more hydrolysis probe(s) according to the present invention, and
- detecting and/or measuring the signal generated by the hydrolysis of the one or more hydrolysis probe(s).

Preferably, the invention relates to a method for the detection of a SNP in a nucleic acid target sequence, wherein in the absence of said SNP from the nucleic acid target sequence essentially no signal generated by the hydrolysis of the one or more hydrolysis probe(s) is detected.

In the context of the present invention, the term "essentially no signal" refers to the signal generated during RT-PCR analysis for the detection of a SNP in a nucleic acid target sequence, wherein said signal may be a determined Ct-value and/or the determined endpoint fluorescent value determined at the end of the RT-PCR run.

In quantitative RT-PCR, the Ct (threshold cycle) value is the intersection between an exponential amplification curve of the fluorescent signal of a sample and a threshold line. It is a relative measure of the concentration of target in the PCR reaction. In case of a Ct-value, the term "essentially no signal" preferably refers to a Ct-value that cannot be determined, because the fluorescent signal that is generated for the respective sample is at no time point increasing exponentially and/or in general is too low to reach the threshold line. In other preferred embodiments, a Ct-value corresponding to essentially "no signal" is at least 7, preferably 8, 9, 10, 12, 14, 16, 18 or 20 cycles higher than a Ct-value determined for a sample that comprises less than 100 copies of the target sequence with the respective SNP and that is otherwise identical to the tested sample. In general, a sample that generates essentially no signal preferably does not generate an exponential fluorescent amplification signal, so that no reliable Ct-value can be determined.

When referring to the absolute amount of fluorescent generated, "essentially no signal" refers to an endpoint fluorescent value that is less than 20 %, preferably less than 15, 14, 13, 12, 11, 10, 9, 8, 7, 6, 5, 4, 3, 2, 1, 0.5, 0.4, 0.3, 0.2, 0.1 % of the fluorescent value of a sample that comprises at least 100 copies of the target sequence with the respective SNP and that is otherwise identical to the tested sample.

In preferred embodiments of the present invention, no specific software is required for determining the presence or absence of a SNP to be detected in a target sequence. In further preferred embodiments, the results of the SNP analysis can be interpreted without the comparison to a positive control sample comprising said SNP or a negative control sample that does not comprise said SNP.

It is a great advantage of the present invention that for the analysis of the result no specific software that compares the result to a positive and a negative control is required. Accordingly, the probes of the invention are advantageous compared to probes that do not comprise said one or more mismatches. If one uses classical probes that are identical to the target sequence with exception of the nucleotide corresponding to the respective SNP, it is required that the signal generated in the sample to be analyzed is compared to a positive control sample comprising said target sequence with said SNP and/or a negative control sample comprising the target sequence, but only without said SNP. When using classical probes, the presence or absence of said SNP in the sample to be analyzed can only be determined on the basis of such a comparison, preferably with the aid of a specific software.

In contrast, the probe of the present invention enables analysis and interpretation of the result of the RT-PCR SNP-analysis without the use of a control samples or a specific analysis software, because in the absence of the SNP essentially no signal is generated. Accordingly, in preferred embodiments the absence of a signal or the absence of a determinable Ct-value indicates the absence of said SNP.

Furthermore, the invention relates to a method for the detection of a SNP in a nucleic acid target sequence, wherein in the absence of said SNP from the nucleic acid target sequence the hydrolysis probe according to the present invention does not anneal with the nucleic acid target sequence.

In another embodiment of the invention the method for the detection of a SNP in a nucleic acid target sequence relates to a SNP in the nucleic acid target sequence, which has been introduced by bisulfite treatment of the sample comprising the nucleic acid sequence and successive amplification of the nucleic acid target sequence.

In a further embodiment of the invention the method for the detection of a SNP in a nucleic acid target sequence comprises the use of one or more pairs of probes according to the present invention.

In another embodiment of the invention the method for the detection of a SNP in a nucleic acid target sequence comprises performing a multiplex PCR.

A further aspect of the invention relates to a kit for the detection of a SNP in a nucleic acid target sequence, **comprising**
a) at least one probe according to the present invention, wherein said probe anneals within the target nucleic acid sequence bound by the oligonucleotide primers of part (i), and
b) a set of oligonucleotide primers for performing a real-time PCR for the amplification of a region of the nucleic acid target sequence comprising the SNP to be detected, wherein each oligonucleotide primer is selected to anneal to its complementary template upstream of any probe of part (a) annealed to the same nucleic acid strand.

Additionally, the invention relates to a kit for the detection of a SNP in a nucleic acid target sequence via real-time PCR, comprising
a) at least one hydrolysis probe according to the invention, or at least one pair of hydrolysis probes according to the invention, wherein at least one hydrolysis probe anneals within a nucleic acid target sequence comprising said SNP, and
b) a set of oligonucleotide primers for performing a real-time PCR for the amplification of a region comprising the nucleic acid target sequence, wherein each oligonucleotide primer is selected to anneal to its complementary template upstream of any probe of part (a) annealed to the same nucleic acid strand.

### DETAILED DESCRIPTION OF THE INVENTION

The present invention is directed to a probe for use in a nucleic acid amplification reaction for the detection of a SNP in a nucleic acid target sequence. In the context of the present invention, the term "probe" or "hydrolysis probe" refers to a hybridization probe, which is a molecule for the detection of a nucleic acid target sequence, which comprises a nucleic acid sequence. It can be used in samples containing nucleic acids to detect the presence of a nucleotide or target sequence that is at least partially complementary to the sequence in the probe, for example by real-time PCR or qRT-PCR. The probe thereby hybridizes to single-stranded nucleic acid whose base sequence allows probe-target base pairing due to complementarity between the probe and target. For detection the probe is labeled (or "tagged") with one or more molecular markers of either radioactive and/or fluorescent and/or a quencher molecules. Commonly used markers are 32P (a radioactive isotope of phosphorus incorporated into the phosphodiester bond in the probe DNA) or Digoxigenin, which is a non-radioactive, antibody-based marker. Detection of sequences with moderate or high similarity depends on how stringent the hybridization conditions were applied - high stringency, such as high hybridization temperature and low salt in hybridization buffers, permits only hybridization between nucleic acid sequences that are highly similar, whereas low stringency, such as lower temperature and high salt, allows hybridization when the sequences are less similar. During RT-PCR, hydrolysis probes comprising a fluorescent marker and a corresponding quencher can be used for detection of a target molecule. During amplification of a target sequence to which a hydrolysis probe is binding by means of primers that lie outside the target sequence, the amplifying polymerase can degrade or hydrolyze the probe from the 5'-end. This leads to the separation of the fluorescence marker and the corresponding quencher that were initially localized in close proximity to each other attached to the probe. Through separation of the fluorescent marker and the quencher, a detectable change in a signal can be generated. Hydrolysis probes are also called 5'-exonuclease probes or TaqMan probes and are well established molecular biology tools that are well known to the person skilled in the art.

The term "nucleic acids" refers to nucleic acid molecules including, without limitation, DNA, ssDNA, dsDNA, RNA, mRNA, tRNA, IncRNA, ncRNA, microRNA, siRNA, rRNA, sgRNA, piRNA, rmRNA, snRNA, snoRNA, scaRNA, gRNA or viral RNA.

The term "nucleic acid amplification reaction" refers to any method comprising an enzymatic reaction, which allows the amplification of nucleic acids. One preferred embodiment of the invention relates to a polymerase chain reaction (PCR). Another preferred embodiment relates to real time PCR (RT-PCR) or quantitative RT-PCR (qRT-PCR), as it allows the quantification of the amplified target in real-time. The term "real-time PCR" is intended to mean any amplification technique which makes it possible to monitor the progress of an ongoing amplification reaction as it occurs (i.e. in real time). Data is therefore collected during the exponential phase of the PCR reaction, rather than at the end point as in conventional PCR. Measuring the kinetics of the reaction the early phases of PCR provides distinct advantages over traditional PCR detection. In real-time PCR, reactions are characterized by the point in time during cycling when amplification of a target is first detected rather than the amount of target accumulated after a fixed number of cycles. The higher the starting copy number of the nucleic acid target, the sooner a significant increase in fluorescence is observed. Traditional PCR methods may also be applied, and use separation methods, such as agarose gels, for detection of PCR amplification at the final phase of or end-point of the PCR reaction. For qRT-PCR no post-PCR processing of the unknown DNA sample is necessary as the quantification occurs in real-time during the reaction. Furthermore, an increase in reporter fluorescent signal is directly proportional to the number of amplicons generated.

Real-time PCR technique can be classified by the chemistry used to detect the PCR product, specific or non-specific fluorochromes. A non-specific DNA-binding dye binds to all double-stranded (ds) DNA in PCR, causing fluorescence of the dye. An increase in DNA product during PCR therefore leads to an increase in fluorescence intensity measured at each cycle.

Specific detection of PCR production can be achieved by using fluorescent reporter probes, which detect only the DNA containing the sequence complementary to the probe. Therefore, use of the reporter probe significantly increases specificity, and enables performing the technique even in the presence of other dsDNA. Using different-colored labels, fluorescent probes can be used in multiplex assays for monitoring several target sequences in the same tube. The specificity of fluorescent reporter probes also prevents interference of measurements caused by primer dimers, which are undesirable potential by-products in PCR. The method relies on a DNA-based probe with a fluorescent reporter at one end and a quencher of fluorescence at the opposite end of the probe. The close proximity of the reporter to the quencher prevents detection of its fluorescence; breakdown of the probe through hydrolysis by the 5' to 3' exonuclease activity of the polymerase used for the amplification reaction breaks the reporter-quencher proximity and thus allows unquenched emission of fluorescence, which can be detected after excitation with a laser. An increase in the product targeted by the reporter probe at each PCR cycle therefore causes a proportional increase in fluorescence due to the breakdown of the probe and release of the reporter. Due to the fact that this kind of assay is based on the 5'-exonuclease activity of the polymerase it is also called "5'-exonuclease assay".

In another preferred embodiment the invention relates to a method, wherein the amplification is a multiplex PCR with more than one pair of primers. The multiplex PCR is a variant of the standard PCR in which two or more loci are simultaneously amplified in the same reaction, by including more than one pair of primers in the reaction.

In the context of the present invention, the term "SNP" refers to any single nucleotide difference between two sequences, e. g. a target sequence with a SNP and a reference sequence. The SNP is preferably a single nucleotide polymorphism as commonly referred to in scientific literature, or to any point mutation in comparison to a defined reference sequence of the nucleic acid target sequence. A single nucleotide polymorphism can be a variation in a single nucleotide that occurs at a specific position in the genome, where each variation is present to some appreciable degree within a population (e.g. >1%).

For example, at a specific base position in the human genome, the base C may appear in most individuals, but in a minority of individuals, the position is occupied by base A. There is a single nucleotide polymorphism at this specific base position, and the two possible nucleotide variations - C or A - are said to be alleles for this base position. For most single nucleotide polymorphisms so far discovered there are only two different alleles, there are also triallelic SNPs in which three different base variations may coexist within a population. Single nucleotide polymorphisms underlie differences in our susceptibility to disease; a wide range of human diseases, e.g. sickle-cell anemia, β-thalassemia and cystic fibrosis result from single nucleotide polymorphisms. The severity of illness and the way our body responds to treatments can be manifestations of genetic variations such as single nucleotide polymorphisms.

Single nucleotide polymorphisms may fall within coding sequences of genes, non-coding regions of genes, or in the intergenic regions (regions between genes). SNPs within a coding sequence do not necessarily change the amino acid sequence of the protein that is produced, due to degeneracy of the genetic code. Single nucleotide polymorphisms in the coding region are of two types, synonymous and nonsynonymous single nucleotide polymorphisms. Synonymous single nucleotide polymorphisms do not affect the protein sequence while nonsynonymous single nucleotide polymorphisms change the amino acid sequence of protein. The nonsynonymous single nucleotide polymorphisms are of two types: missense and nonsense. Single nucleotide polymorphisms that are not in protein-coding regions may still affect gene splicing, transcription factor binding, messenger RNA degradation, or the sequence of non-coding RNA. Gene expression affected by this type of single nucleotide polymorphism is referred to as an eSNP (expression SNP) and may be upstream or downstream from the gene.

A point mutation, or single base modification, refers to a type of mutation that causes a single nucleotide base substitution of the genetic material including DNA and RNA. Point mutations are single nucleotide polymorphism (SNP) mutations in a nucleic acid and usually take place during nucleic acid replication. Point mutations may arise from spontaneous mutations that occur during nucleic acid replication. The mutation may be caused by a mutagen. The rate of mutation may be increased by mutagens. Mutagens can be physical, such as radiation from UV rays, X-rays or extreme heat, or chemical (molecules that misplace base pairs or disrupt the helical shape of DNA). Mutagens associated with cancers are often studied to learn about cancer and its prevention. Point mutations can occur in response to environmental challenges. They are more likely to occur when they are advantageous to the organism, rather than when they are neutral or disadvantageous. When cells were deprived of a certain amino acid, tryptophan, for prolonged periods of time, point mutations in trp operon reverted to tryptophan, leading to an advantageous result, more frequently than under normal conditions when the mutations were neutral. In addition, the tryptophan mutation rate was unaffected when the cells were deprived of another amino acid, cysteine, further suggesting that the mutation rate was specific to situations in which the mutation was advantageous.

Point mutations that occur in non-coding sequences are often without consequences, although there are exceptions. If the mutated base pair is in the promoter sequence of a gene, then the expression of the gene may change. Also, if the mutation occurs in the splicing site of an intron, then this may interfere with correct splicing of the transcribed pre-mRNA. Point germline mutations can lead to beneficial as well as harmful traits or diseases. This leads to adaptations based on the environment where the organism lives. An advantageous mutation can create an advantage for that organism and lead to the trait's being passed down from generation to generation, improving and benefiting the entire population. On the other hand, harmful mutations cause the organism to die or be less likely to reproduce in a phenomenon known as natural selection. Other effects of point mutations, or single nucleotide polymorphisms in DNA, depend on the location of the mutation within the gene. For example, if the mutation occurs in the region of the gene responsible for coding, the amino acid sequence of the encoded protein may be altered, causing a change in the function, activation, localization, or stability of the protein. Moreover, if the mutation occurs in the region of the gene where transcriptional machinery binds, the mutation can affect the binding of the transcription factors because the short nucleotide sequences recognized by the transcription factors will be altered. Mutations in this region can affect rate of efficiency of gene transcription, which in turn can alter levels of mRNA and, thus, protein levels in general.

Point mutations can have several effects on the behavior and reproduction of a protein depending on where the mutation occurs in the amino acid sequence of the protein. If the mutation occurs in the region of the gene that is responsible for coding for the protein, the amino acid may be altered. This slight change in the sequence of amino acids can cause a change in the function, activation of the protein meaning how it binds with a given enzyme, where the protein will be located within the cell, or the amount of free energy stored within the protein. If the mutation occurs in the region of the gene where transcriptional machinery binds to the protein, the mutation can affect the way in which transcription factors bind to the protein. The mechanisms of transcription bind to a protein through recognition of short nucleotide sequences. A mutation in this region may alter these sequences and, thus, change the way the transcription factors bind to the protein. Mutations in this region can affect the efficiency of gene transcription, which controls both the levels of mRNA and overall protein levels.

Point mutations can be introduced into a nucleic acid sequence by bisulfite treatment of the sample comprising the nucleic acid sequence, which converts cytosine residues to uracil, but leaves 5-methylcytosine residues unaffected, and successive amplification of the nucleic acid target sequence, which will lead to introduction of thymidine at the position of the un-methylated cytosine, which then represents a point mutation in comparison to the reference sequence.

The term "target sequence" refers to the nucleic acid sequence that is to be detected by the probe. The nucleic acid target sequence comprises a nucleic acid sequence, which is at least partially complementary to the nucleic acid sequence comprised in the probe and comprises the respective SNP that is to be detected by the probe.

A probe according to the present invention comprises an oligonucleotide sequence complementary to the region of the nucleic target acid sequence comprising said SNP. Said oligonucleotide sequence of the probe comprises, consists or essentially consists of 5 to 100 bases, preferably 5 to 50, 10 to 40, 12 to 38, 13 to 35, 14 to 32, 15 to 28, 16 to 26, 17 to 25, 18 to 24, 19 to 23, 20 to 22, 21 to 22, or most preferably 22 bases. The length of the probe is therefore preferably between 10 and 40 nucleotides in length, more preferably 10, 11 12, 13, 14, 15, 16, 17, 18, 19, 20, 21, 22, 23, 24, 25, 26, 27, 28, 29, 30, 31, 32, 34, 3,5 36, 37, 38, 39, or 40 nt.

A probe according to the present invention further comprises a pair of interactive labels, at least one label being signal-generating and the labels being effectively positioned on the oligonucleotide to quench the generation of detectable signal, said labels being separated by a nuclease susceptible cleavage site. The term "label" as used herein refers to any atom or molecule which can be used to provide a detectable (preferably quantifiable) signal, and which can be attached to a nucleic acid or protein. Labels may provide signals detectable by fluorescence, radioactivity, colorimetry, gravimetry, X-ray diffraction or absorption, magnetism, enzymatic activity, and the like.

The oligonucleotide is labeled by incorporating moieties detectable by spectroscopic, photochemical, biochemical, immunochemical, or chemical means. The method of linking or conjugating the label to the oligonucleotide probe depends, of course, on the type of label(s) used and the position of the label on the probe. A variety of labels that would be appropriate for use in the invention, as well as methods for their inclusion in the probe, are known in the art and include, but are not limited to, enzymes (e.g., alkaline phosphatase and horseradish peroxidase) and enzyme substrates, radioactive atoms, fluorescent dyes, chromophores, chemiluminescent labels, electrochemiluminescent labels, such as OrigenTM (Igen), ligands having specific binding partners, or any other labels that may interact with each other to enhance, alter, or diminish a signal. Of course, should the PCR be practiced using a thermal cycler instrument, the label must be able to survive the temperature cycling required in this automated process.

Among radioactive atoms, 32p is preferred. Methods for introducing 32P into nucleic acids are known in the art, and include, for example, 5' labeling with a kinase, or random insertion by nick translation. Enzymes are typically detected by their activity. "Specific binding partner" refers to a protein capable of binding a ligand molecule with high specificity, as for example in the case of an antigen and a monoclonal antibody specific therefor. Other specific binding partners include biotin and avidin or streptavidin, IgG and protein A, and the numerous receptor-ligand couples known in the art. The above description is not meant to categorize the various labels into distinct classes, as the same label may serve in several different modes. For example, ¹²⁵I may serve as a radioactive label or as an electron-dense reagent. HRP may serve as enzyme or as antigen for a monoclonal antibody. Further, one may combine various labels for desired effect. For example, one might label a probe with biotin, and detect the presence of the probe with avidin labeled with ¹²⁵I, or with an antibiotin monoclonal antibody labeled with HRP. Other permutations and possibilities will be readily apparent to those of ordinary skill in the art and are considered as equivalents within the scope of the instant invention.

The term "pair of interactive labels" refers to two labels, which influence each other in a way that a signal generated by at least one of the labels is influenced, modified or changed by the other label, so that the resulting overall signal generated by the probe is quenched, which implies deletion, depletion or lowering of the signal generated by at least one of the labels. Therefore, at least one of the labels must be signal-generating. "Effectively positioned" means that the pair of interactive labels is positioned in a way, that they can interact with each other. So the distance between the two labels should be appropriate for the kind of interaction that is occurring. The labels may be attached to the oligonucleotide directly or indirectly by a variety of techniques. Such techniques are well established and known to the one skilled in the art and therefore do not require further explanation. Depending on the precise type of label used, the label can be located at the 5' or 3' end of the probe, located internally in the probe, or attached to spacer arms of various sizes and compositions to facilitate signal interactions.

The term "nuclease" refers to an enzyme capable of cleaving the phosphodiester bonds between the nucleotide subunits of nucleic acids. Nucleases comprise endonucleases, exonucleases with 5' and 3' exonuclease activity, and site-specific nucleases. A restriction endonuclease functions by "scanning" the length of a DNA molecule. Once it encounters its particular specific recognition sequence, it will bind to the DNA molecule and makes one cut in each of the two sugar-phosphate backbones. The positions of these two cuts, both in relation to each other, and to the recognition sequence itself, are determined by the identity of the restriction endonuclease. Exonucleases are enzymes that work by cleaving nucleotides one at a time from the end (exo) of a polynucleotide chain. A hydrolyzing reaction that breaks phosphodiester bonds at either the 3' or the 5' end occurs. Nuclease susceptible cleavage sites comprise phosphodiester bonds between nucleotide subunits of a nucleic acid.

Generally the 3' terminus of the probe will be "blocked" to prohibit incorporation of the probe into a primer extension product. "Blocking" can be achieved by using non-complementary bases or by adding a chemical moiety such as biotin or a phosphate group to the 3' hydroxyl of the last nucleotide, which may, depending upon the selected moiety, serve a dual purpose by also acting as a label for subsequent detection or capture of the nucleic acid attached to the label. Blocking can also be achieved by removing the 3'-OH or by using a nucleotide that lacks a 3'-OH such as a dideoxynucleotide. "Primer extension product" refers to a nucleic acid molecule which results from an oligonucleotide or primer, which has been extended by a polymerase through the attachment of nucleotides to its 3'-end.

The term "mismatch" refers to the presence of a base that would lead to an uncomplimentary base pairing, or lack of base pairing, in double-stranded nucleic acid molecule. In the context of the present invention, the probe according to the present invention is complementary to the respective target sequence, wherein it comprises one or more bases within its oligonucleotide sequence, which are not complementary to the corresponding base of the target sequence and therefore represent a mismatch upon hybridization of the probe to its target sequence.

In a preferred embodiment of the present invention, one label is a fluorophore and the other label is a quencher which interacts with said fluorophore.

A "fluorophore" (or fluorochrome, similarly to a chromophore) is a fluorescent chemical compound that can re-emit light upon light excitation. Fluorophores for use as labels in constructing labeled probes of the invention comprise, without claiming to be exhaustive, rhodamine and derivatives, such as Texas Red, fluorescein and derivatives, such as 5-bromomethyl fluorescein, Lucifer Yellow, IAEDANS, 7-Me2N-coumarin-4-acetate, 7-OH-4-CH3-coumarin-3-acetate, 7-NH2-4CH3-coumarin-3-acetate (AMCA), monobromobimane, pyrene trisulfonates, such as Cascade Blue, and monobromotrimethyl-ammoniobimane, FAM, TET, CAL Fluor Gold 540, HEX, JOE, VIC, CAL Fluor Orange 560, Cy3, NED, Quasar 570, Oyster 556, TMR, CAL Fluor Red 590, ROX, LC red 610, CAL Fluor Red 610, Texas red, LC red 610, CAL Fluor Red 610, LC red 640, CAL Fluor Red 635, Cy5, LC red 670, Quasar 670, Oyster 645, LC red 705, Cy5.5, BODIPY FL, Oregon Green 488, Rhodamine Green, Oregon Green 514, Cal Gold, BODIPY R6Gj, Yakima Yellow, JOE, HEX, Cal Orange, BODIPY TMR-X, Quasar-570 /Cy3, TAMRA, Rhodamine Red-X, Redmond Red, BODIPY 581/591, Cy3.5, Cal Red/Texas Red, BODIPY TR-X, BODIPY 630/665-X, Pulsar-650, Quasar-670/Cy5.

"Quenching" refers to any process which decreases the fluorescence intensity of a given substance. Quenching is the basis for Förster resonance energy transfer (FRET) assays. FRET is a dynamic quenching mechanism because energy transfer occurs while the donor is in the excited state. A "quencher" is a molecule, which quenches the fluorescence emitted by the fluorophore when excited by the cycler's light source via FRET. Quenchers for use as labels in constructing labeled probes of the invention comprise, without claiming to be exhaustive, DDQ-I, Dabcyl, Eclipse, Iowa Black FQ, BHQ-1, QSY-7, BHQ-2, DDQ-II, Iowa Black RQ, QSY-21, BHQ-3, QSY-35, BHQ-0, QSY-9, ElleQuencher, Iowa Black. The one skilled in the art can choose suitable reporter-quencher pairs, as this has been described in the literature *[*Johansson, M.K. Methods in Molecular Biology 335, 17 - 29 (2006*);* Marras, S.A. Methods in Molecular Biology 335, 3-16 (2006*)].*

The term "microorganism" refers to a living organism so small in size that it is only visible with the aid of a microscope, comprising bacteria, archaea, protozoa, fungi, algae, and viruses.

The term "mammal" refers to all mammals including humans. Mammals comprise, without claiming to be exhaustive, humans, non-human primates, cows, dogs, cats, goats, sheep, pigs, rats, mice and rabbits.

The term "activating mutation" refers to mutations that lead to a gain-of-function changing the gene product such that its effect gets stronger (enhanced activation) or even is superseded by a different and abnormal function. In mammals and humans, such mutations are often associated with diseases including cancer, and detection of such mutations is highly relevant for diagnosis and decision-making concerning treatment options.

Bisulfite sequencing is a method for the determination of the methylation pattern of DNA, wherein DNA is undergoing bisulfite treatment, which converts cytosine residues to uracil, but leaves 5-methylcytosine residues unaffected. Therefore, DNA that has been treated with bisulfite retains only methylated cytosines. Thus, bisulfite treatment introduces specific changes in the DNA sequence that depend on the methylation status of individual cytosine residues, yielding single-nucleotide resolution information about the methylation status of a segment of DNA.

The term "bisulfite" refers to a bisulfite ion (IUPAC-recommended nomenclature: hydrogen sulfite), which has the formula HSO₃⁻. Salts containing the HSO₃⁻ ion are termed bisulfites.

The invention further relates to all sequences according to Table 1.

**Table 1. Preferred sequences of the present invention. SNPs are marked in bold and mismatches are marked in italics and underlined.**

| **SEQ ID No.** | **Probe ID** | **Description** | **Sequence (5'-3')** |
|---|---|---|---|
| 1 | 7416 | CD95L, methylation | AGTATAG**C**GATAGTAATT*C*AGGTTTTGA |
| 2 | 6912 | rs2032583 T-Allele | CTGATT**A**GAATACTTTACTCCA*C*TTAATTAA |
| 3 | 6950 | rs2032583 C-Allele | ATTC**C**AATCAGTGTTATTT*C*GTTACTC |
| 4 | 7023 | rs2235015 G-Allele | CATA**C**CATTTATGTCTCTTT*C*GTCTC |
| 5 | 7128 | rs2235015 T-Allele | AAATG**T**TATGTTTGTTT*C*GTGGTG |
| 6 | 5899 | TEM-39 | CGT**C**CACCCAACT*T*ATCTTCAG |
| 7 | 6773 | TEM240 | CCGGT**A**AGCGTGG*A*TCTCG |
| 8 | 7433 | HFE, C282Y, C-Allele | ACCTGG*C*ACGTA**A**ATCTCTGC |
| 9 | 7543 | HFE, C282Y, -Allele | ATACGT*A*CCAGGTG**C**AGCACC |
| 10 | 7196 | IDH1, C-Allele | CATGA*C*GACCTATGATG**C**TAGGTTT |
| 11 | 7197 | IDH1, T-Allele | TAGGTC*T*TCATGCTTA**C**GGGGA |
| 12 | 7625 | Factor II - Mutant allele | AGCA**A**GCCTCAA*C*GCTCC |
| 13 | 7371 | Factor II - WT allele | AGGCT**C**GCTGAGAG*C*CACTT |
| 14 | 7626 | Factor V - Mutant allele | AGGC**A**AGGAATACAGG*C*ATTT |
| 15 | 7432 | Factor V - WT allele | TTCCT**C**GCCTGTCCA*A*GGA |

The term "a pair of probes" refers to two probes according to the present invention, which are to be used in the same reaction for the detection of a certain SNP or the respective reference sequence, so that the presence and quantity of nucleic acid molecules containing the respective target sequences can be determined from the same reaction.

The term "semiquantitative method" refers to a method for quantification, which is based on an internal reference sequence to determine fold-difference in abundance of the reference sequence in comparison to the target sequence.

The term "quantitative method" refers to a method for quantification, which aims to determine the exact number of nucleic acid target molecules by comparison with a nucleic acid standard using a calibration curve.

### FIGURES

The invention is further described by the following figures. These are not intended to limit the scope of the invention, but represent preferred embodiments of aspects of the invention provided for greater illustration of the invention described herein.

### Brief description of the figures:

**Figure 1****:** Real-time PCR assay for the detection of factor II mutations.
**Figure 2****:** Real-time PCR assay for the detection of factor V mutations.
**Figure 3****:** Real-time PCR assay for the detection of a mutation of the beta-lactamase-gene TEM-39 inducing ESBL (extended spectrum beta-lactamases).
**Figure 4****:** Real-time PCR assay for the detection of a methylated C in the human gene encoding CD95L on bisulfite-treated DNA.

### Detailed description of the figures:

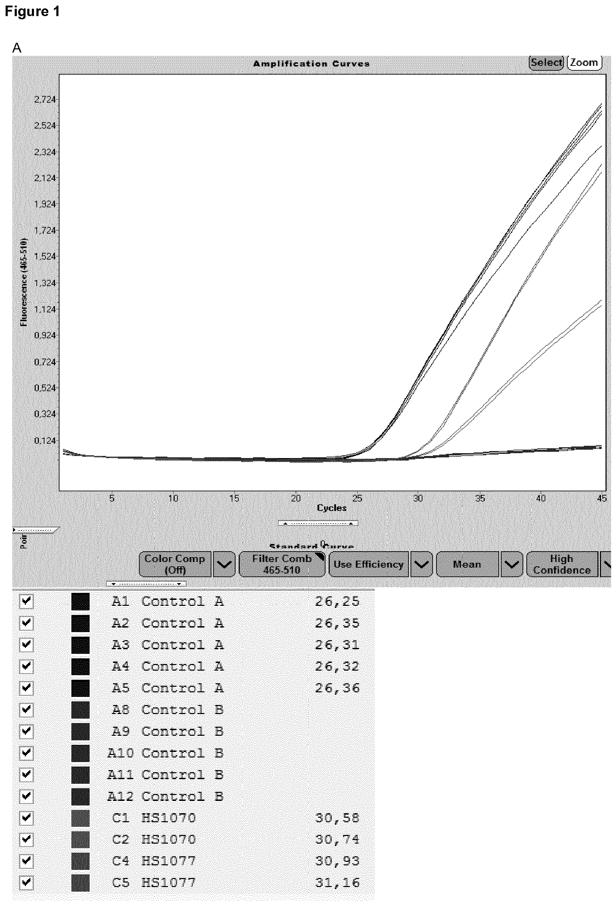

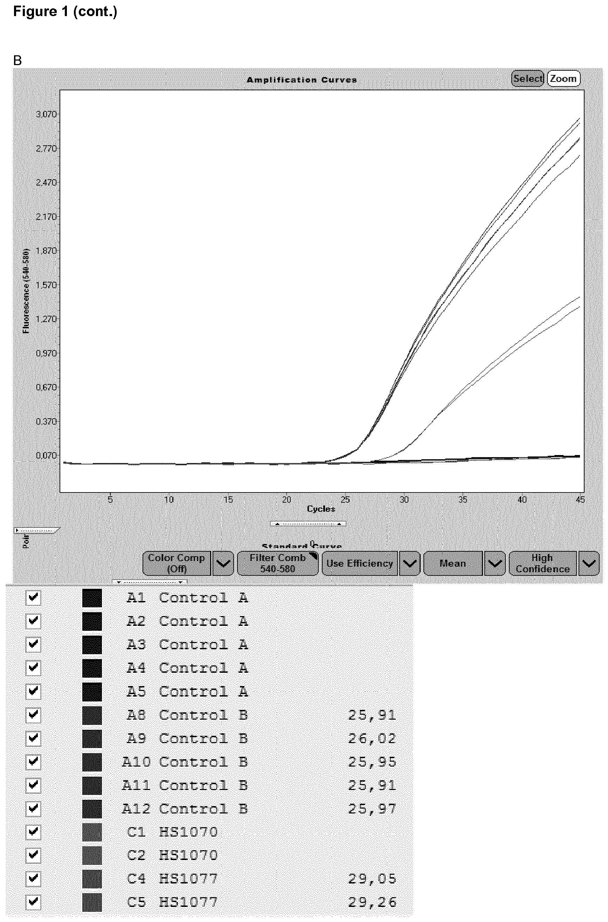

**Figure 1****:** Real time PCR assay for the detection of WT factor II (**A**) in the FAM channel and mutant factor II (**B**) in the VIC channel in one PCR reaction. Samples containing nucleic acid sequences encoding WT and/or mutant factor II are analyzed by real-time PCR using factor II specific primers and probes according to the present invention, wherein the probes are specific for the WT or the mutant form of factor II. The WT probe comprises a FAM label and the mutant probe comprises a VIC label. Blue line: homozygous WT. Red line: homozygous WT. Purple line: Heterozygous WT/mutant. Green line: homozygous mutant.

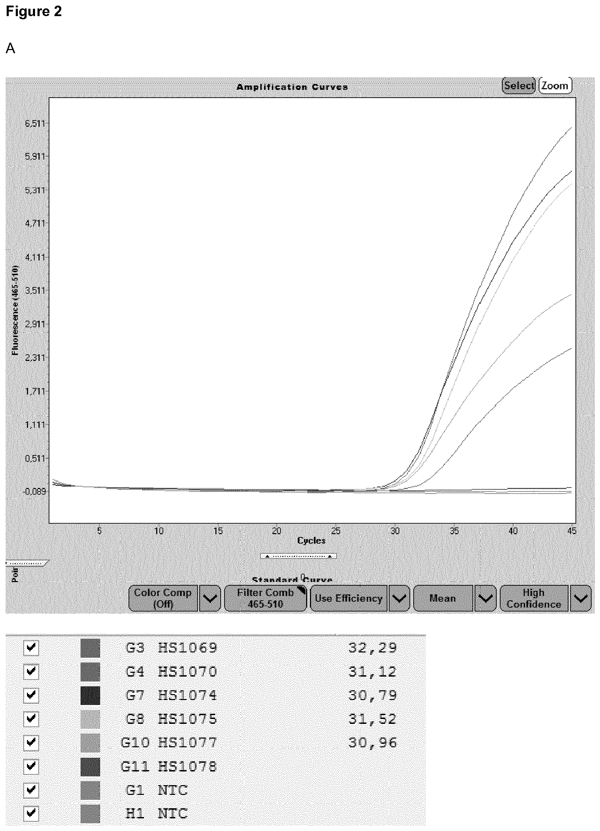

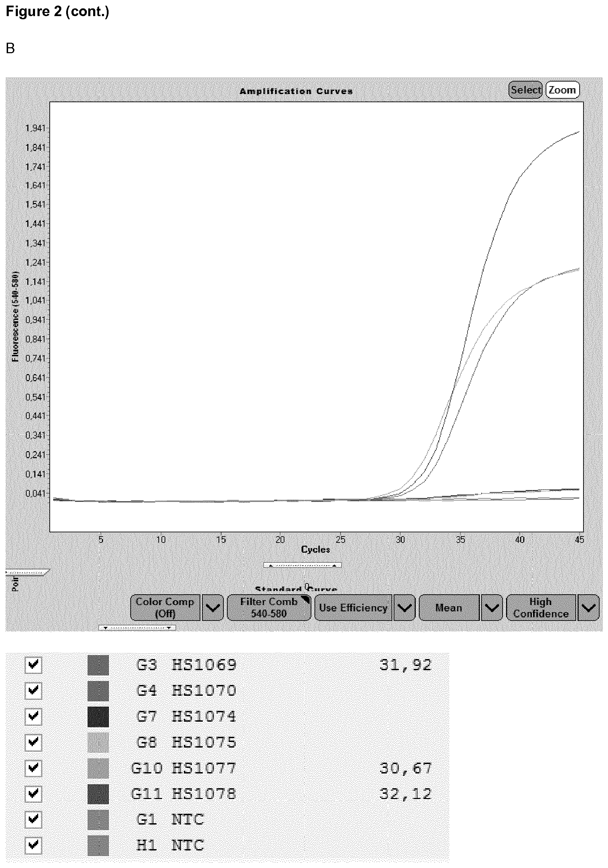

**Figure 2****:** Real time PCR assay for the detection of WT factor V (**A**) in the FAM channel and mutant factor V (**B**) in the VIC channel in one PCR reaction. Samples containing nucleic acid sequences encoding WT and/or mutant factor V are analyzed by real-time PCR using factor V specific primers and probes according to the present invention, wherein the probes are specific for the WT or the mutant form of factor V. The WT probe comprises a FAM label and the mutant probe comprises a VIC label. Turquois, pink and red lines: homozygous WT. Blue and orange lines: Heterozygous WT/mutant. Purple line: homozygous mutant.

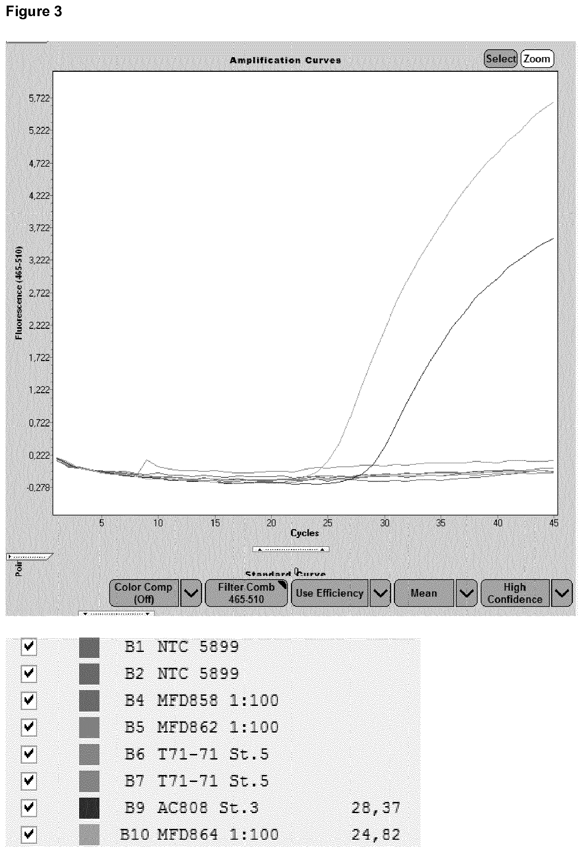

**Figure 3****:** Real-time PCR assay for the detection of a mutation of the beta-lactamase-gene TEM-39 inducing ESBL (extended spectrum beta-lactamases) using different samples. Blue line: no template nucleic acid (negative control). Green line: WT gene. Light green, grey and pink lines: WT TEM-39. Dark green and orange lines: mutant TEM-39.

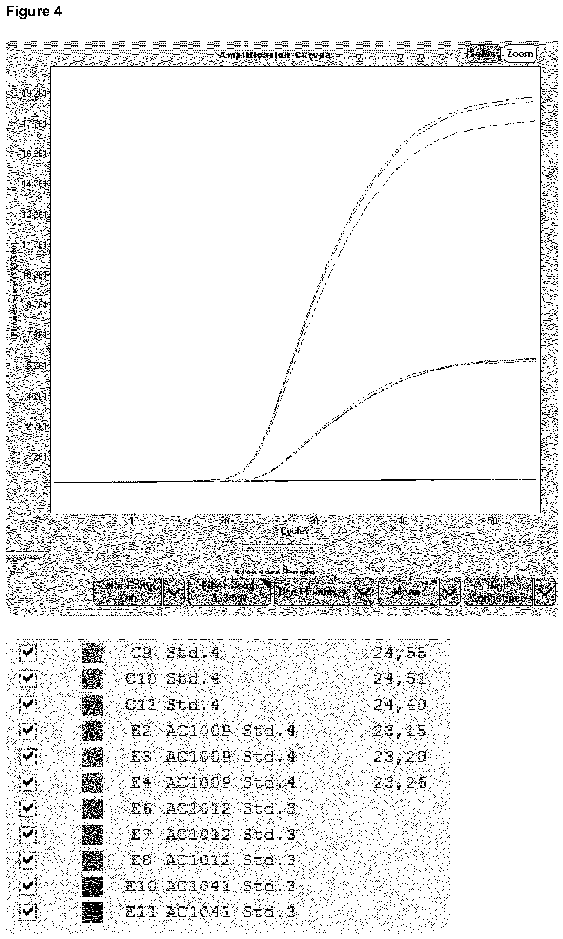

**Figure 4****:** Real-time PCR assay for the detection of a mutation of a methylated C in the human gene encoding CD95L on bisulfite-treated DNA. Pink line: 100% methylated. Blue line: 30 % methylated, 70% non-methylated. Purple line: 100% non-methylated. Black line: non bisulfite treated DNA, 0% converted.

### EXAMPLES

The invention is further described by the following examples. These are not intended to limit the scope of the invention, but represent preferred embodiments of aspects of the invention provided for greater illustration of the invention described herein.

**Description of the probes listed in Table 1** SEQ ID No. 1 (AGTATAGCGATAGTAATTCAGGTTTTGA) is specific for the human gene encoding CD95L and detects a methylated C on bisulfite-treated DNA. A probe according to the present invention comprising or consisting of SEQ ID No. 1 can be used in a companion diagnostic for a glioblastoma medicament.

The present invention therefore relates to a method comprising the use of the probe as described herein. The method may further comprise diagnosis of a medical conditions, or therapy control of a particular ongoing treatment.

SEQ ID No. 2 (CTGATTAGAATACTTTACTCCACTTAATTAA) and SEQ ID No. 3 (ATTCCAATCAGTGTTATTTCGTTACT) are specific for the rs2032583 T-allele and C-allele of the human gene encoding the ABCB1-transporter, respectively. SEQ ID No. 4 (CATACCATTTATGTCTCTTTCGTCTC) and SEQ ID No. 5 (AAATGTTATGTTTGTTTCGTGGTG) are specific for the rs2235015 G-allele and T-allele of the human gene encoding the ABCB1-transporter, respectively. Probes according to the present invention comprising or consisting of SEQ ID No. 2 to 5 can be used in a companion diagnostics to detect SNP-variants. The four different probes can be used in a single 4-plex PCR reaction, if coupled to distinguishable fluorophore/quencher pairs.

SEQ ID No. 6 (CGTCCACCCAACTTATCTTCAG) and SEQ ID No. 7 (CCGGTAAGCGTGGATCTCG) are specific for the beta-lactamase-genes TEM-39 and TEM-240, respectively, and probes according to the present invention comprising or consisting of SEQ ID No. 6 or 7 can be used for detecting mutations inducing ESBL (extended spectrum beta-lactamases) by real-time PCR.

SEQ ID No. 8 (ACCTGGCACGTAAATCTCTGC) and SEQ ID No. 9 (ATACGTACCAGGTGCAGCACC) are specific for the C-allele and the A-allele of the human *HFE*-gene, respectively. Two different probes according to the present invention, one comprising or consisting of SEQ ID No. 8 and the other comprising or consisting of SEQ ID No. 9, which are labeled with distinguishable fluorophore/quencher pairs, can be used in a single real-time PCR reaction for the detection of the C282Y mutation in the nucleic acid target sequence to diagnose haemochromatosis, which is induced by this point mutation.

SEQ ID No. 10 (CATGACGACCTATGATGCTAGGTTT) and SEQ ID No. 11 (TAGGTCTTCATGCTTACGGGGA) are specific for the C-allele and the T-allele of the human *IDH1-gene,* respectively. Two different probes according to the present invention, one comprising or consisting of SEQ ID No. 10 and the other comprising or consisting of SEQ ID No. 11, which are labeled with distinguishable fluorophore/quencher pairs, can be used in a single real-time PCR reaction for the detection of a cancer-relevant point mutation in the IDH1-gene. Such probes can be useful for the development of companion diagnostics.

SEQ ID No. 12 (AGCAAGCCTCAACGCTCC) and SEQ ID No. 13 (AGGCTCGCTGAGAGCCACTT) are specific for the mutant and the WT allele of the human *factor II*-gene (gene encoding the clotting factor II, also called prothrombin), respectively. Two different probes according to the present invention, one comprising or consisting of SEQ ID No. 12 and the other comprising or consisting of SEQ ID No. 13, which are labeled with distinguishable fluorophore/quencher pairs, can be used in a single real-time PCR reaction for the detection of factor II mutations associated with coagulation problems and/or blood clotting.

SEQ ID No. 14 (AGGCAAGGAATACAGGCATTT) and SEQ ID No. 15 (TTCCTCGCCTGTCCAAGGA) are specific for the mutant and the WT allele of the human *factor V*-gene (gene encoding the clotting factor II, also called prothrombin), respectively. Two different probes according to the present invention, one comprising or consisting of SEQ ID No. 14 and the other comprising or consisting of SEQ ID No. 15, which are labeled with distinguishable fluorophore/quencher pairs, can be used in a single real-time PCR reaction for the detection of factor V mutations associated with Factor V Leiden thrombophilia.

The above mentioned preferred sequences of SEQ ID NO 1-15 have been employed in corresponding methods used to detect SNPs in samples. The corresponding probes, which represent using different targets, different SNPs and different positioning of the mismatches within the probes, have all lead to preliminary promising results with respect to the desired outcome, namely the generation of signal in an amplification reaction when the SNP is present (i. e. when only one mismatch occurs between the probe and the target sequence) and the production of negligent (or no) signal when the SNP is absent (i. e. when two (or more) mismatches between probe and target are evident).

### Methods employed in the Examples

### Real-time PCR protocol for detection of a SNP:

The components used in the real-time PCR were in the concentrations and volumes as specified in Table 2.

**Table 2. Composition of real-time PCR reaction mix.**

| **Component** | **Volume in µl** |
|---|---|
| Water | 10,3 |
| 10 x PCR buffer | 2 |
| Deoxyribonucleotide triphosphates mixture, concentration: 2,5mM | 2 |
| MgCl₂, concentration: 25mM | 4 |
| Primers 0,25 µl, concentration 20pmol/µl | 1,0 |
| Probes 0,06 µl, concentration: 20pmol/µl | 0,6 |
| TAQ-Polymerase, concentration: 10U/µl | 0,1 |
| Total Volume master mix | 20 |
| DNA sample/extract | 5 |
| **Total Volume** | **25** |

The following PCR profile was used for performing the SNP detection reaction:

**Table 3. Temperature profile of real-time PCR reaction according to the present invention.**

| **Reaction step** | **Temperature** | **Time** |
|---|---|---|
| Initial denaturation | 95°C | 1 min |
| Denaturation | 95°C | 10 sec |
| Annealing/extension | 60°C | 15 sec |

45 Cycles of Denaturation and Annealing/extension were performed.

### Example 1

The real-time PCR protocol for detection of a SNP as detailed above was performed for the detection of WT and mutant factor II in a sample.

The following primers were used for amplification of the factor II gene:

| | |
|---|---|
| Primer No. 7460: | AACCAATCCCGTGAAAGAATTA |
| Pimer No. 7461: | AGAGCTGCCCATGAATAGCA |

The following probes were included in the real-time PCR reaction:

| | |
|---|---|
| Probe No. 7625 | AGCAAGCCTCAACGCTCC (SEQ ID No. 12, mutant allele of factor II, FAM labeled) |
| Probe No. 7371 | AGGCTCGCTGAGAGCCACTT (SEQ ID No. 13, WT allele of factor II, VIC labeled) |

Samples containing nucleic acid sequences encoding WT and/or mutant factor II were analyzed by real-time PCR. Signals were detected in the FAM and VIC channels, leading to the results displayed in Table 4 and Figure 1.

**Table 4. Results of real-time PCR analysis for the detection of WT and mutant factor II.**

| **Sample** | **Color-code** | **Replicate** | **Ct FAM channel** | **Ct VIC channel** |
|---|---|---|---|---|
| WT homozygous | Blue | 1 | 26,25 | n.d. |
| WT homozygous | Blue | 2 | 26,35 | n.d. |
| WT homozygous | Blue | 3 | 26,31 | n.d. |
| WT homozygous | Blue | 4 | 26,32 | n.d. |
| WT homozygous | Blue | 5 | 26,36 | n.d. |
| WT homozygous | Red | 1 | 30,58 | n.d. |
| WT homozygous | Red | 2 | 30,74 | n.d. |
| WT/Mutant heterozygous | Purple | 1 | 30,93 | 25,91 |
| WT/Mutant heterozygous | Purple | 2 | 31,16 | 26,02 |
| Mutant homozygous | Green | 1 | n.d. | 25,95 |
| Mutant homozygous | Green | 2 | n.d. | 25,91 |
| Mutant homozygous | Green | 3 | n.d. | 25,97 |
| Mutant homozygous | Green | 4 | n.d. | 29,05 |
| Mutant homozygous | Green | 5 | n.d. | 29,26 |

The probe specific for WT factor II only generates a signal in the FAM channel for samples that contain nucleic acid sequences encoding WT factor II. The probe specific for mutant factor II only generates a signal in the VIC channel for samples that contain nucleic acid sequences encoding mutant factor II.

### Example 2

The real-time PCR protocol for detection of a SNP as detailed above was performed for the detection of WT and mutant factor V in a sample.

The following primers were used for amplification of the factor II gene:

| | |
|---|---|
| Primer No. 7067: | CTTCTAATCTGTAAGAGCAGATCCC |
| Pimer No. 7068: | GGAGACCTAACATGTTCTAGCCA |

The following probes were included in the real-time PCR reaction:

| | |
|---|---|
| Probe No. 7626 | AGGCAAGGAATACAGGCATTT (SEQ ID No. 14, mutant allele of factor V, FAM labeled) |
| Probe No. 7432 | TTCCTCGCCTGTCCAAGGA (SEQ ID No. 15, WT allele of factor V, VIC labeled) |

Samples containing nucleic acid sequences encoding WT and/or mutant factor V were analyzed by real-time PCR. Signals were detected in the FAM and VIC channels, leading to the results displayed in Table 5 and Figure 2.

**Table 5. Results of real-time PCR analysis for the detection of WT and mutant factor V.**

| **Sample** | **Calor-code** | **Replicate** | **Ct FAM channel** | **Ct VIC channel** |
|---|---|---|---|---|
| WT homozygous | Turquois | 1 | 31,52 | n.d. |
| WT homozygous | Pink | 2 | 31,12 | n.d. |
| WT homozygous | Red | 3 | 30,79 | n.d. |
| WT/Mutant heterozygous | Blue | 1 | 32,29 | 31,92 |
| WT/Mutant heterozygous | Orange | 2 | 30,96 | 30,67 |
| Mutant homozygous | Purple | 1 | n.d. | 32,12 |
| No sample (negative control) | Green | 1 | n.d. | n.d. |
| No sample (negative control) | Green | 2 | n.d. | n.d. |

The probe specific for WT factor V only generates a signal in the FAM channel for samples that contain nucleic acid sequences encoding WT factor II. The probe specific for mutant factor II only generates a signal in the VIC channel for samples that contain nucleic acid sequences encoding mutant factor V.

### Example 3

The real-time PCR protocol for detection of a SNP as detailed above was performed for the detection of WT and mutant TEM-39 in a sample.

The following primers were used for amplification of the factor II gene:

| | |
|---|---|
| Primer No. 4889: | TTCCTGTTTTTGCTCACCCA |
| Pimer No. 4890: | GATCCAGTTCGATGTAACCCA |

The following probe was included in the real-time PCR reaction:

| | |
|---|---|
| Probe No. 5899 | CGTCCACCCAACTTATCTTCAG (SEQ ID No. 7, detecting TEM-39) |

Samples containing nucleic acid sequences that are negative and positive for mutant TEM-39 were analyzed by real-time PCR. Signals were detected in the FAM channel, leading to the results displayed in Table 6 and Figure 3.

**Table 6. Results of real-time PCR analysis for the detection of TEM-39.**

| **Sample** | **Calor-code** | **Replicate** | **Ct FAM channel** |
|---|---|---|---|
| No sample (negative control) | Blue | 1 | n.d. |
| No sample (negative control) | Blue | 2 | n.d. |
| WT TEM-39 | Pink | 1 | n.d. |
| WT TEM-39 | Grey | 2 | n.d. |
| WT | Green | 1 | n.d. |
| WT | Green | 2 | n.d. |
| Mutant TEM-39 | Dark green | 1 | 28,37 |
| Mutant TEM-39 | Orange | 2 | 24,82 |

The probe specific for mutant TEM-39 only generates a signal in the FAM channel for samples that contain nucleic acid sequences encoding mutant TEM-39.

### Example 4

The real-time PCR protocol for detection of a SNP detailed above was performed for the detection of methylated CD95L in a sample.

The following primers were used for amplification of the factor II gene:

| | |
|---|---|
| Primer No. 5041: | GGATTTTAGGAAGGTGAGTATAGTTTA |
| Pimer No. 6169: | AAATTTCCGCCCACAATTT |

The following probe was included in the real-time PCR reaction:

| | |
|---|---|
| Probe No. 7416: | AGTATAGCGATAGTAATTCAGGTTTTGA (SEQ ID No. 1, detecting methylated CD95L) |

Samples containing nucleic acid sequences encoding for CD95L with different grades of methylation were analyzed by real-time PCR after bisulfite-treatment. Signals were detected in the FAM channel, leading to the results displayed in Table 7 and Figure 4.

**Table 7. Results of real-time PCR analysis for the detection of methylated CD95L.**

| **Sample** | **Color-code** | **Replicate** | **Ct FAM channel** |
|---|---|---|---|
| 100% methylated | Pink | 1 | 23,15 |
| 100% methylated | Pink | 2 | 23,2 |
| 100% methylated | Pink | 3 | 23,26 |
| 30 % methylated, 70% non-methylated | Blue | 1 | 24,55 |
| 30 % methylated, 70% non-methylated | Blue | 1 | 24,51 |
| 30 % methylated, 70% non-methylated | Blue | 1 | 24,4 |
| 100% non-methylated | Purple | 1 | n.d. |
| 100% non-methylated | Purple | 2 | n.d. |
| 100% non-methylated | Purple | 3 | n.d. |
| WT, not treated with bisulfite | Black | 1 | n.d. |
| WT, not treated with bisulfite | Black | 1 | n.d. |

The probe specific for the methylated CD95L gene only generates a signal in the FAM channel for samples that contain nucleic acid sequences that contained methylated CD95L.

## Claims

1. A hydrolysis probe for use in a real-time PCR for the detection of a SNP in a nucleic acid target sequence, said probe comprising
a) an oligonucleotide sequence complementary to a region of a nucleic acid target sequence comprising said SNP, and
b) a pair of interactive labels, at least one label being signal-generating and the labels being effectively positioned on the oligonucleotide to quench the generation of detectable signal, said labels being separated by a nuclease susceptible cleavage site,
c) wherein said probe is blocked at the 3'-end terminus to prohibit incorporation of said probe into a primer extension product, and
**wherein** said oligonucleotide sequence of said probe comprises two adjacent portions, said portions being of equal length or differing by one nucleotide in length, wherein
- the base complementary to said SNP is located within the first portion of the probe sequence from the 5'-end, and
- located within the second portion of the probe there is one or more mismatches to the target sequence,
**or**
- the base complementary to said SNP is located within the second portion of the probe, and
- located within the first portion of the probe there is one or more mismatches to the target sequence.

2. A hydrolysis probe according to claim 1, **wherein** said one or more mismatches to the target sequence are C-A, C-T or A-A mismatches, preferably C-A or C-T mismatches.

3. A hydrolysis probe according to any one of the preceding claims, **wherein**
a) the base complementary to said SNP is located within a central part of said first portion, wherein said central part consists essentially of a sequence comprising half of the nucleotides of the first portion, and
b) said one or more mismatches are located within a central part of said second portion, wherein said central part consists essentially of a sequence comprising half of the nucleotides of the second portion,
or
c) the base complementary to said SNP is located within a central part of said second portion, wherein said central part consists essentially of a sequence comprising half of the nucleotides of the second portion, and
d) said one or more mismatches are located within a central part of said first portion, wherein said central part consists essentially of a sequence comprising half of the nucleotides of the first portion.

4. A hydrolysis probe according to any one of the preceding claims, **wherein** the probe has a length of 16 to 32 nucleotides, preferentially 18 to 24 nucleotides.

5. A hydrolysis probe according to any one of the preceding claims, **wherein** one label is a fluorophore and the other label is a quencher, which interacts with said fluorophore.

6. A hydrolysis probe according to any one of the preceding claims, **wherein** said nucleic acid target sequence is obtained and/or derived from a microorganism or from a mammal, wherein the SNP is preferably an activating mutation.

7. A hydrolysis probe according to any one of the preceding claims, **wherein** the SNP in the nucleic acid target sequence has been introduced by bisulfite treatment of the sample comprising the nucleic acid sequence and successive amplification of the nucleic acid target sequence.

8. A hydrolysis probe according to any one of the preceding claims, **wherein** the oligonucleotide sequence of said probe comprises, consists or essentially consists of any one of the oligonucleotide sequences SEQ ID No. 1 to 15.

9. A pair of hydrolysis probes for use in a real-time PCR for the detection of a SNP in a nucleic acid target sequence, **wherein**
a) a first hydrolysis probe is a probe according to any one of claims 1 to 8, and
b) a second hydrolysis probe is a probe according to any one of claims 1 to 8 comprising the complementary oligonucleotide sequence as the first probe, with the exception that in place of the base that is complementary to the SNP, the second probe comprises a base that is complementary to the corresponding base of a nucleic acid target sequence that does not comprise said SNP, and
c) the signals obtained from the fluorophores of the two probes can be distinguished from each other, preferably through a semi-quantitative or quantitative method.

10. A method for the detection of a SNP in a nucleic acid target sequence comprising:
a) amplification of a region comprising the nucleic acid target sequence comprising the SNP by real-time PCR employing one or more reporter probe(s),
wherein the one or more reporter probe(s) comprise one or more hydrolysis probe(s) according to claims 1 to 8, and
b) detecting and/or measuring the signal generated by the hydrolysis of the one or more hydrolysis probe(s).

11. A method for the detection of a SNP in a nucleic acid target sequence according to claim 10, **wherein** in the absence of said SNP from the nucleic acid target sequence essentially no signal generated by the hydrolysis of the one or more hydrolysis probe(s) is detected.

12. A method for the detection of a SNP in a nucleic acid target sequence according to any of claims 10 or 11, **wherein** in the absence of said SNP from the nucleic acid target sequence the hydrolysis probe according to claims 1 to 8 does not anneal with the nucleic acid target sequence.

13. A method for the detection of a SNP in a nucleic acid target sequence according to any of claims 10 to 12, **wherein** the SNP in the nucleic acid target sequence has been introduced by bisulfite treatment of the sample comprising the nucleic acid sequence and successive amplification of the nucleic acid target sequence.

14. A method for the detection of a SNP in a nucleic acid target sequence according to any one of claims 10 to 13, **wherein** said method comprises the use of one or more pairs of probes according to claim 9, and/or wherein said method comprises performing a multiplex PCR.

15. A kit for the detection of a SNP in a nucleic acid target sequence via real-time PCR, **comprising**
a) at least one hydrolysis probe according to claims 1 to 8, or at least one pair of hydrolysis probes according to claim 9, wherein at least one hydrolysis probe anneals within a nucleic acid target sequence comprising said SNP, and
b) a set of oligonucleotide primers for performing a real-time PCR for the amplification of a region comprising the nucleic acid target sequence, wherein each oligonucleotide primer is selected to anneal to its complementary template upstream of any probe of part (a) annealed to the same nucleic acid strand.

## Patentansprüche

1. Hydrolysesonde zur Verwendung in einer Echtzeit-PCR zur Detektion eines SNP in einer Nukleinsäure-Zielsequenz, wobei die Sonde Folgendes umfasst
a) eine Oligonukleotidsequenz, die zu einer Region einer Nukleinsäure-Zielsequenz komplementär ist, die den SNP umfasst, und
b) ein Paar interaktiver Markierungen, wobei mindestens eine Markierung signalerzeugend ist und die Markierungen effektiv auf dem Oligonukleotid positioniert sind, um die Erzeugung eines detektierbaren Signals zu quenchen, wobei die Markierungen durch eine für Nukleasen empfängliche Spaltstelle getrennt sind,
c) wobei die Sonde an dem 3'-terminalen Ende blockiert ist, um den Einbau der Sonde in ein Primer-Extensions-Produkt zu verhindern, und
wobei die Oligonukleotidsequenz der Sonde zwei benachbarte Abschnitte umfasst, wobei die Abschnitte die gleiche Länge aufweisen oder sich in der Länge um ein Nukleotid unterscheiden, wobei
- die zu dem SNP komplementäre Base innerhalb des ersten Abschnitts der Sondensequenz von dem 5'-Ende liegt, und
- innerhalb des zweiten Abschnitts der Sonde eine oder mehrere Fehlpaarungen mit der Zielsequenz liegen,
oder
- die zu dem SNP komplementäre Base innerhalb des zweiten Abschnitts der Sonde liegt, und
- innerhalb des ersten Abschnitts der Sonde eine oder mehrere Fehlpaarungen mit der Zielsequenz liegen.

2. Hydrolysesonde nach Anspruch 1, wobei die eine oder mehreren Fehlpaarungen mit die Zielsequenz C-A-, C-T- oder A-A-Fehlpaarungen sind, vorzugsweise C-A- oder C-T-Fehlpaarungen.

3. Hydrolysesonde nach einem der vorhergehenden Ansprüche, wobei
a) die zu dem SNP komplementäre Base innerhalb eines zentralen Teils des ersten Abschnitts liegt, wobei der zentrale Teil im Wesentlichen aus einer Sequenz besteht, die die Hälfte der Nukleotide des ersten Abschnitts umfasst, und
b) die eine oder mehreren Fehlpaarungen innerhalb eines zentralen Teils des zweiten Abschnitts liegt/liegen, wobei der zentrale Teil im Wesentlichen aus einer Sequenz besteht, die die Hälfte der Nukleotide des zweiten Abschnitts umfasst,
oder
c) die zu dem SNP komplementäre Base innerhalb eines zentralen Teils des zweiten Abschnitts liegt, wobei der zentrale Teil im Wesentlichen aus einer Sequenz besteht, die die Hälfte der Nukleotide des zweiten Abschnitts umfasst, und
d) die eine oder mehreren Fehlpaarungen innerhalb eines zentralen Teils des ersten Abschnitts liegt/liegen, wobei der zentrale Teil im Wesentlichen aus einer Sequenz besteht, die die Hälfte der Nukleotide des ersten Abschnitts umfasst.

4. Hydrolysesonde nach einem der vorhergehenden Ansprüche, wobei die Sonde eine Länge von 16 bis 32 Nukleotiden, vorzugsweise 18 bis 24 Nukleotiden aufweist.

5. Hydrolysesonde nach einem der vorhergehenden Ansprüche, wobei eine Markierung ein Fluorophor und die andere Markierung ein Quencher ist, der mit dem Fluorophor interagiert.

6. Hydrolysesonde nach einem der vorhergehenden Ansprüche, wobei die Nukleinsäure-Zielsequenz von einem Mikroorganismus oder von einem Säugetier erhalten und/oder gewonnen wird, wobei der SNP vorzugsweise eine aktivierende Mutation ist.

7. Hydrolysesonde nach einem der vorhergehenden Ansprüche, wobei der SNP in der Nukleinsäure-Zielsequenz durch Bisulfit-Behandlung der Probe, die die Nukleinsäuresequenz umfasst, und sukzessive Amplifikation der Nukleinsäure-Zielsequenz eingefügt wurde.

8. Hydrolysesonde nach einem der vorhergehenden Ansprüche, wobei die Oligonukleotidsequenz der Sonde eine beliebige der Oligonukleotidsequenzen SEQ ID No. 1 bis 15 umfasst, aus dieser besteht oder im Wesentlichen besteht.

9. Paar Hydrolysesonden zur Verwendung in einer Echtzeit-PCR zur Detektion eines SNP in einer Nukleinsäure-Zielsequenz, wobei
a) eine erste Hydrolysesonde eine Sonde nach einem der Ansprüche 1 bis 8 ist und
b) eine zweite Hydrolysesonde eine Sonde nach einem der Ansprüche 1 bis 8 ist, die die komplementäre Oligonukleotidsequenz als erste Sonde umfasst, mit der Ausnahme, dass die zweite Sonde anstelle der zum SNP komplementären Base eine Base umfasst, die komplementär zu der entsprechenden Base einer Nukleinsäure-Zielsequenz ist, die den SNP nicht umfasst, und
c) die von den Fluorophoren der beiden Sonden erhaltenen Signale voneinander unterschieden werden können, vorzugsweise durch ein semi-quantitatives oder quantitatives Verfahren.

10. Verfahren zur Detektion eines SNP in einer Nukleinsäure-Zielsequenz, umfassend:
a) Amplifikation einer Region, die die Nukleinsäure-Zielsequenz umfasst, die den SNP umfasst, durch Echtzeit-PCR unter Verwendung einer oder mehrerer Reportersonde(n),
wobei die eine oder die mehreren Reportersonde(n) eine oder mehrere Hydrolysesonde(n) gemäß den Ansprüchen 1 bis 8 umfasst/umfassen, und
b) Detektieren und/oder Messen des Signals, das durch die Hydrolyse der einen oder mehreren Hydrolysesonde(n) erzeugt wird.

11. Verfahren zur Detektion eines SNP in einer Nukleinsäure-Zielsequenz nach Anspruch 10, wobei bei Abwesenheit des SNP aus der Nukleinsäure-Zielsequenz im Wesentlichen kein Signal detektiert wird, das durch die Hydrolyse der einen oder mehreren Hydrolysesonde(n) erzeugt wird.

12. Verfahren zur Detektion eines SNP in einer Nukleinsäure-Zielsequenz nach einem der Ansprüche 10 oder 11, wobei bei Abwesenheit des SNP aus der Nukleinsäure-Zielsequenz sich die Hydrolysesonde nach den Ansprüchen 1 bis 8 nicht mit der Nukleinsäure-Zielsequenz verbindet.

13. Verfahren zur Detektion eines SNP in einer Nukleinsäure-Zielsequenz nach einem der Ansprüche 10 bis 12, wobei der SNP in der Nukleinsäure-Zielsequenz durch eine Bisulfitbehandlung der Probe, die die Nukleinsäuresequenz umfasst, und sukzessive Amplifikation der Nukleinsäure-Zielsequenz, eingefügt wurde.

14. Verfahren zur Detektion eines SNP in einer Nukleinsäure-Zielsequenz nach einem der Ansprüche 10 bis 13, wobei das Verfahren die Verwendung eines oder mehrerer Sondenpaare nach Anspruch 9 umfasst und/oder wobei das Verfahren das Durchführen einer Multiplex-PCR umfasst.

15. Kit zur Detektion eines SNP in einer Nukleinsäure-Zielsequenz mittels Echtzeit-PCR, umfassend
a) mindestens eine Hydrolysesonde nach Anspruch 1 bis 8 oder mindestens ein Paar Hydrolysesonden nach Anspruch 9, wobei sich mindestens eine Hydrolysesonde innerhalb einer Nukleinsäure-Zielsequenz, die das SNP umfasst, verbindet und
b) einen Satz von Oligonukleotid-Primern zum Durchführen einer Echtzeit-PCR zur Amplifikation einer Region, die die Nukleinsäure-Zielsequenz umfasst, wobei jeder Oligonukleotid-Primer ausgewählt ist, um sich mit seiner komplementären Matrize stromaufwärts einer Sonde von Teil (a), die mit demselben Nukleinsäurestrang verbunden ist, zu verbinden.

## Revendications

1. Sonde d'hydrolyse à utiliser dans un PCR en temps réel pour la détection d'un SNP dans une séquence cible d'acide nucléique, ladite sonde comprenant
a) une séquence oligonucléotidique complémentaire d'une région d'une séquence cible d'acide nucléique comprenant ledit SNP, et
b) une paire d'étiquettes interactives, au moins une étiquette étant génératrice de signal et les étiquettes étant positionnées efficacement sur l'oligonucléotide pour désactiver la génération de signal détectable, lesdites étiquettes étant séparées par un site de coupure sensible à la nucléase,
c) dans laquelle ladite sonde est bloquée au terminus d'extrémité 3' pour empêcher l'incorporation de ladite sonde dans un produit d'extension d'amorce, et
dans laquelle ladite séquence oligonucléotidique de ladite sonde comprend deux parties adjacentes, lesdites parties étant de longueur égale ou différant d'un nucléotide en longueur, dans laquelle
- la base complémentaire dudit SNP est située à l'intérieur de la première partie de la séquence de sonde par rapport à l'extrémité 5', et
- situés à l'intérieur de la seconde partie de la sonde se trouvent un ou plusieurs mésappariements avec la séquence cible,
ou
- la base complémentaire dudit SNP est située à l'intérieur de la seconde partie de la sonde, et
- situés à l'intérieur de la première partie de la sonde se trouvent un ou plusieurs mésappariements avec la séquence cible.

2. Sonde d'hydrolyse selon la revendication 1, dans laquelle lesdits un ou plusieurs mésappariements avec la séquence cible sont des mésappariements C-A, C-T ou A-A, de préférence des mésappariements C-A ou C-T.

3. Sonde d'hydrolyse selon l'une quelconque des revendications précédentes, dans laquelle
a) la base complémentaire dudit SNP est située à l'intérieur d'une partie centrale de ladite première partie, dans laquelle ladite partie centrale se compose fondamentalement d'une séquence comprenant la moitié des nucléotides de la première partie, et
b) lesdits un ou plusieurs mésappariements sont situés à l'intérieur d'une partie centrale de ladite seconde partie, dans laquelle ladite partie centrale se compose fondamentalement d'une séquence comprenant la moitié des nucléotides de la seconde partie,
ou
c) la base complémentaire dudit SNP est située à l'intérieur d'une partie centrale de ladite seconde partie, dans laquelle ladite partie centrale se compose fondamentalement d'une séquence comprenant la moitié des nucléotides de la seconde partie, et
d) lesdits un ou plusieurs mésappariements sont situés à l'intérieur d'une partie centrale de ladite première partie, dans laquelle ladite partie centrale se compose fondamentalement d'une séquence comprenant la moitié des nucléotides de la première partie.

4. Sonde d'hydrolyse selon l'une quelconque des revendications précédentes, dans laquelle la sonde a une longueur de 16 à 32 nucléotides, de préférence de 18 à 24 nucléotides.

5. Sonde d'hydrolyse selon l'une quelconque des revendications précédentes, dans laquelle une étiquette est un fluorophore et l'autre étiquette est un désactiveur, qui interagit avec ledit fluorophore.

6. Sonde d'hydrolyse selon l'une quelconque des revendications précédentes, dans laquelle ladite séquence cible d'acide nucléique est obtenue à partir d'un micro-organisme ou d'un mammifère, ou dérivée de ceux-ci, dans laquelle le SNP est de préférence une mutation activatrice.

7. Sonde d'hydrolyse selon l'une quelconque des revendications précédentes, dans laquelle le SNP dans la séquence cible d'acide nucléique a été introduit par un traitement bisulfite de l'échantillon comprenant la séquence d'acide nucléique et une amplification successive de la séquence cible d'acide nucléique.

8. Sonde d'hydrolyse selon l'une quelconque des revendications précédentes, dans laquelle la séquence oligonucléotidique de ladite sonde comprend, se compose de ou se compose fondamentalement de l'une quelconque des séquences oligonucléotidiques SEQ ID No. 1 à 15.

9. Paire de sondes d'hydrolyse à utiliser dans un PCR en temps réel pour la détection d'un SNP dans une séquence cible d'acide nucléique, dans laquelle
a) une première sonde d'hydrolyse est une sonde selon l'une quelconque des revendications 1 à 8, et
b) une seconde sonde d'hydrolyse est une sonde selon l'une quelconque des revendications 1 à 8 comprenant la séquence oligonucléotidique complémentaire en tant que première sonde, excepté le fait qu'à la place de la base qui est complémentaire du SNP, la seconde sonde comprend une base qui est complémentaire de la base correspondante d'une séquence cible d'acide nucléique qui ne comprend pas ledit SNP, et
c) les signaux obtenus à partir des fluorophores des deux sondes peuvent être distingués entre eux, de préférence à travers un procédé semi-quantitatif ou quantitatif.

10. Procédé pour la détection d'un SNP dans une séquence cible d'acide nucléique comprenant :
a) l'amplification d'une région comprenant la séquence cible d'acide nucléique comprenant le SNP par un PCR en temps réel utilisant une ou plusieurs sondes rapporteuses,
dans lequel les une ou plusieurs sondes rapporteuses comprennent une ou plusieurs sondes d'hydrolyse selon les revendications 1 à 8, et
b) la détection et/ou la mesure du signal généré par l'hydrolyse des une ou plusieurs sondes d'hydrolyse.

11. Procédé pour la détection d'un SNP dans une séquence cible d'acide nucléique selon la revendication 10, dans lequel, en l'absence dudit SNP de la séquence cible d'acide nucléique, fondamentalement aucun signal généré par l'hydrolyse des une ou plusieurs sondes d'hydrolyse n'est détecté.

12. Procédé pour la détection d'un SNP dans une séquence cible d'acide nucléique selon l'une quelconque des revendications 10 ou 11, dans lequel, en l'absence dudit SNP de la séquence cible d'acide nucléique, la sonde d'hydrolyse selon les revendications 1 à 8 ne s'hybride pas avec la séquence cible d'acide nucléique.

13. Procédé pour la détection d'un SNP dans une séquence cible d'acide nucléique selon l'une quelconque des revendications 10 à 12, dans lequel le SNP dans la séquence cible d'acide nucléique a été introduit par un traitement bisulfite de l'échantillon comprenant la séquence d'acide nucléique et une amplification successive de la séquence cible d'acide nucléique.

14. Procédé pour la détection d'un SNP dans une séquence cible d'acide nucléique selon l'une quelconque des revendications 10 à 13, dans lequel ledit procédé comprend l'utilisation d'une ou plusieurs paires de sondes selon la revendication 9, et/ou dans lequel ledit procédé comprend la réalisation d'un PCR multiplex.

15. Kit pour la détection d'un SNP dans une séquence cible d'acide nucléique via un PCR en temps réel, comprenant
a) au moins une sonde d'hydrolyse selon les revendications 1 à 8, ou au moins une paire de sondes d'hydrolyse selon la revendication 9, dans lequel au moins une sonde d'hydrolyse s'hybride à l'intérieur d'une séquence cible d'acide nucléique comprenant ledit SNP, et
b) un ensemble d'amorces oligonucléotidiques pour réaliser un PCR en temps réel pour l'amplification d'une région comprenant la séquence cible d'acide nucléique, dans lequel chaque amorce oligonucléotidique est sélectionnée pour s'hybrider à sa matrice complémentaire en amont de toute sonde de la partie (a) hybridée au même brin d'acide nucléique.


### SEQUENCE LISTING

<110> Mergemeier Steffen
<120> PROBE FOR DETECTION OF SNPs
<130> 2147/16WO
<160> 23
<170> BiSSAP 1.3.6
<210> 1
   <211> 28
   <212> DNA
   <213> Homo sapiens
<400> 1
   agtatagcga tagtaattca ggttttga 28
<210> 2
   <211> 31
   <212> DNA
   <213> Homo sapiens
<220>
   <223> rs2032583 T-Allele
<400> 2
   ctgattagaa tactttactc cacttaatta a 31
<210> 3
   <211> 27
   <212> DNA
   <213> Homo sapiens
<220>
   <223> rs2032583 C-Allele
<400> 3
   attccaatca gtgttatttc gttactc 27
<210> 4
   <211> 26
   <212> DNA
   <213> Homo sapiens
<220>
   <223> rs2235015 G-Allele
<400> 4
   cataccattt atgtctcttt cgtctc 26
<210> 5
   <211> 24
   <212> DNA
   <213> Homo sapiens
<220>
   <223> rs2235015 T-Allele
<400> 5
   aaatgttatg tttgtttcgt ggtg 24
<210> 6
   <211> 22
   <212> DNA
   <213> Homo sapiens
<220>
   <223> TEM-39
<400> 6
   cgtccaccca acttatcttc ag 22
<210> 7
   <211> 19
   <212> DNA
   <213> Homo sapiens
<220>
   <223> TEM240
<400> 7
   ccggtaagcg tggatctcg 19
<210> 8
   <211> 21
   <212> DNA
   <213> Homo sapiens
<220>
   <223> HFE, C282Y, C-Allele
<400> 8
   acctggcacg taaatctctg c 21
<210> 9
   <211> 21
   <212> DNA
   <213> Homo sapiens
<220>
   <223> HFE, C282Y, -Allele
<400> 9
   atacgtacca ggtgcagcac c 21
<210> 10
   <211> 25
   <212> DNA
   <213> Homo sapiens
<220>
   <223> IDH1, C-Allele
<400> 10
   catgacgacc tatgatgcta ggttt 25
<210> 11
   <211> 22
   <212> DNA
   <213> Homo sapiens
<220>
   <223> IDH1, T-Allele
<400> 11
   taggtcttca tgcttacggg ga 22
<210> 12
   <211> 18
   <212> DNA
   <213> Homo sapiens
<220>
   <223> Factor II \226 Mutant allele
<400> 12
   agcaagcctc aacgctcc 18
<210> 13
   <211> 20
   <212> DNA
   <213> Homo sapiens
<220>
   <223> Factor II \226 WT allele
<400> 13
   aggctcgctg agagccactt 20
<210> 14
   <211> 21
   <212> DNA
   <213> Homo sapiens
<220>
   <223> Factor V \226 Mutant allele
<400> 14
   aggcaaggaa tacaggcatt t 21
<210> 15
   <211> 19
   <212> DNA
   <213> Homo sapiens
<220>
   <223> Factor V \226 WT allele
<400> 15
   ttcctcgcct gtccaagga 19
<210> 16
   <211> 22
   <212> DNA
   <213> Homo sapiens
<400> 16
   aaccaatccc gtgaaagaat ta 22
<210> 17
   <211> 20
   <212> DNA
   <213> Homo sapiens
<400> 17
   agagctgccc atgaatagca 20
<210> 18
   <211> 25
   <212> DNA
   <213> Homo sapiens
<400> 18
   cttctaatct gtaagagcag atccc 25
<210> 19
   <211> 23
   <212> DNA
   <213> Homo sapiens
<400> 19
   ggagacctaa catgttctag cca 23
<210> 20
   <211> 20
   <212> DNA
   <213> Homo sapiens
<400> 20
   ttcctgtttt tgctcaccca 20
<210> 21
   <211> 21
   <212> DNA
   <213> Homo sapiens
<400> 21
   gatccagttc gatgtaaccc a 21
<210> 22
   <211> 27
   <212> DNA
   <213> Homo sapiens
<400> 22
   ggattttagg aaggtgagta tagttta 27
<210> 23
   <211> 19
   <212> DNA
   <213> Homo sapiens
<400> 23
   aaatttccgc ccacaattt 19